# EUROPEAN PATENT SPECIFICATION

(11) **EP 3 294 766 B1**
(45) Date of publication and mention of the grant of the patent: **09.08.2023**
(21) Application number: 16720842.0
(22) Date of filing: 06.05.2016
(51) Int. Cl.: C07K 16/28, C12N 5/0783

(54) **HUMANIZED ANTIBODY OR FRAGMENT THEREOF SPECIFIC FOR CD3**
HUMANIZED ANTIBODY OR FRAGMENT THEREOF SPECIFIC FOR CD3
ANTICORPS HUMANISÉ OU FRAGMENT DE CELUI-CI SPÉCIFIQUE POUR CD3

(30) Priority: 08.05.2015 EP 15166966
(43) Date of publication of application: 21.03.2018
(73) Proprietor: Miltenyi Biotec B.V. & Co. KG, 51429 Bergisch Gladbach (DE)
(72) Inventor: NÖLLE, Volker, 51515 Kürten (DE); THIE, Holger, 88400 Biberach an der Riss (DE); KAISER, Andrew, 51503 Rösrath (DE)
(74) Representative: Biervert, Christian
(86) International application number: PCT/EP2016/060148
(87) International publication number: WO 2016/180721

(56) References cited:
- EP-A1- 2 711 418
- WO-A1-2013/186613
- WO-A1-2013/188693
- WO-A2-2012/143524

## Description

### Field of invention

The present invention relates to the field of humanized antibodies or fragments thereof, in particular to antibodies or fragments thereof specific for the antigen CD3 and uses thereof.

### Background of the invention

The clone OKT3 is a mouse monoclonal IgG2a antibody specific for the human cell surface marker CD3 (T-cell surface glycoprotein CD3, a signal-transducing co-receptor of the T cell receptor, comprising the subunits gamma, delta, epsilon, and zeta), recognizing an atypically small area of CD3 epsilon. OKT3 was originally described in US4658019 and Kung et al. (1979) Science 206, 347-349. For stimulation of human T cells using anti-CD3 full-length antibodies, OKT3 is well-known for being a potent stimulator of T cell activation and proliferation (US6406696; US6143297; US6113901; Yannelly et al. (1990) J. Immunol. Meth. 130, 91-100). T cell activation by anti-CD3 antibodies involves a complex molecular interaction. Soluble anti-CD3 antibodies were unable to induce the activation of T cells *in vitro* in the absence of Fc receptor-bearing cells; and it has been shown that such activation depends on T-cell receptor (TCR)/CD3 complex cross-linking on the surface of T cells, which was mediated by anti-CD3 antibody OKT3 linking both T cells (via its variable regions) and Fc receptor-bearing cells (via its Fc portion) (Ceuppens et al. (1985) J Immunol. 135, 3882-3886; Bohua et al. (2005) Immunology 116, 487-498). The monovalent Fab fragment of OKT3 is about 100 times less potent than the parent IgG (Van Wauve et al. (1980) J Immunol. 124, 2708-2713).

Geppert and Lipsky (J. Immunol. (1987) 138, 1660-1666) reported the use of immobilized anti-CD3 monoclonal antibodies to induce the proliferation of a purified population of T cells. The authors found that immobilized OKT3 was not very effective in stimulating T cell proliferation. Anti-CD3-antibodies have also been described as agents used in combination with anti-CD28-antibodies to induce T cell proliferation (US6352694). Clones 15E8, CD28.3, and Leu-28 are examples for mouse monoclonal antibodies specific for human CD28 antigen. Anti-CD28 antibodies with distinct binding properties differ in their ability to induce T cell activation (Nunès et al. (1993) Int Immunol. 5, 311-315). In WO9429436A1, an *in vitro*method is disclosed for inducing a population of T cells to proliferate, comprising contacting a population of T cells with a solid phase surface having directly immobilized thereon: (a) a first agent which provides a primary activation signal to the T cells (regularly anti-CD3 antibody), thereby activating the T cells; and (b) a second agent which stimulates an accessory molecule on the surface of the T cells (regularly anti-CD28 antibody), thereby stimulating the activated T cells, the first and second agents thereby inducing the T cells to proliferate. Agents such as anti-CD3 and anti-CD28 antibodies which allow for polyclonal stimulation of T cells may also be attached to a matrix of mobile polymer chains as disclosed in WO2014048920 or EP2711418A1.

T cells can be stimulated to increase the transduction efficiency (for example lentiviral transduction). The transduction efficiency of quiescent T cells is usually very low, because the cell do not express receptors (such as LDL receptor in the case of Vesicular stomatitis virus (VSV) G-protein pseudotyped lentiviral vectors) required for transduction. Stimulation of T cells by anti-CD3 antibody alone or in combination with anti-CD28 antibody results in expression of these receptors and therefore leads to higher transduction efficiencies (Amirache et al. (2014) Blood 123, 1422-1424).

Unfortunately, the murine OKT3 antibody shows a low stability. WO1998052975 discloses a mutated scFv variant of the murine anti-CD3 antibody OKT3 containing an exchange position H100A according to the Kabat numbering system. The mutated OKT3 scFv is produced using a recombinant expression system and WO1998052975 proposes that the mutated anti-CD3 scFv is more stable than the parental OKT3 antibody during extended storage periods.

Humanized antibodies or fragments thereof are useful for clinical and cell therapeutic purposes as they are less immunogenic when administered to human patients compared to non-humanized antibodies or fragments thereof such as murine antibodies or fragments thereof. Techniques for humanization of non-human antibodies are known in the art and comprise complementary determining regions (CDR) grafting into a human or humanized framework, wherein the grafted CDRs are typically derived from a rodent antibody. CDRs are part of the variable regions in antibodies or fragments thereof, where these molecules bind to their specific antigen. They are embedded into framework regions which are much less variable than the CDRs. US6750325, US5885573, US7381803, US20040052783 and US20040202657 disclose the transfer of binding specificity from OKT3 into human frameworks. WO2002051871 discloses humanized antibodies obtained from variable regions of CD28.3, produced by the hybridoma CNCM 1-2582. CD28.3 is almost unable to induce IL-2 release / T cell activation (Nunès et al. (1993) Int Immunol. 5, 311-315).

WO2013188693A1 discloses a construct having a scFv comprising the sequences of the present SEQ ID NO:16 and 17.

WO2012143524A2 discloses bispecific antibodies binding HER2 and CD3. One of the CD3 Fab-arms comprises the sequences of the present SEQ ID NO: 16 and 17.

WO2013186613A1 discloses recombinant humanized anti-CD3 antibodies comprising the CDRs of the present invention. These humanized antibodies are based on OKT3 and the cysteine to serine mutation at position 114 according to IMGT nomenclature in the CDR3 of the heavy chain of OKT3 leads to a significant improvement in the biological activity ans stability of the antibodies.

There is a need in the art for a humanized anti-CD3 antibody or fragment thereof which is more stable than the murine parental OKT3 antibody, and which for example can be used for polyclonal stimulation of T cells either alone or in combination with a humanized anti-CD28 antibody or fragment thereof.

### Summary of the invention

We have surprisingly found that the expression level of CDR-mutated Fab variants of anti-CD3 antibody clone OKT3 was 40- to 70-fold higher than the level of the non-CDR-mutated Fab variant and that the expression level of humanized Fab variants of anti-CD28 antibody clone 15E8 was 10-fold higher than the level of the non-humanized Fab variant, when expressed in *E.coli.* In the case of OKT3, a CDR-mutated and humanized variant showed the highest expression level, wherein the CDR mutation comprises the amino acid substitution cysteine to serine at position 114 according to IMGT nomenclature in the CDR3 of the heavy chain variable domain.

Secondly, the resulting Fab molecules of CDR-mutated OKT3 variants (humanized as well as non-humanized) surprisingly showed an increased stability compared to the non-CDR-mutated Fab variant and to murine parental full-length OKT3 antibody. The increased stability was detected for the free Fab molecule as well for tagged Fab molecules.

Thirdly, it was unexpectedly found that the CDR-mutated OKT3 variants (humanized as well as non-humanized) in Fab format are able to stimulate T cells when bound to a matrix of mobile polymer chains, and even more surprisingly that these are more potent as full-length OKT3 antibody. This was completely against expectation because the cross-linking on the surface of T cells was thought to be mediated not only by the variable regions, but also by the Fc portion of OKT3.

Fourthly, it was unexpectedly found that humanized 15E8 variants in Fab format which are specific for the antigen CD28 are able to stimulate T cells in combination with either murine OKT3 antibody or CDR-mutated OKT3 Fab variants (humanized as well as non-humanized) as least as potent as murine 15E8 antibody when bound to a matrix of mobile polymer chains. Again this was contrary to the expectation because of the missing Fc portion of the Fab. The combination of a humanized and CDR-mutated anti-CD3 Fab variant of OKT3 with a humanized anti-CD28 Fab variant of 15E8, each bound to a matrix of mobile polymer chains, gave the best result in terms of high frequency of activated T cells plus high proliferation rate of T cells.

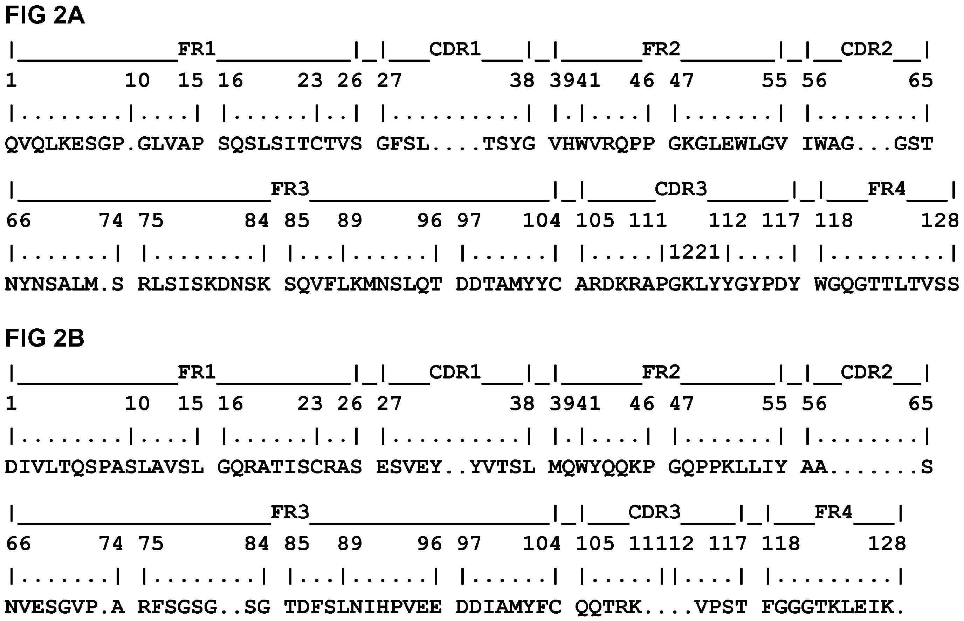

The CDRs (according to IMGT nomenclature) of anti-CD3 antibody clone OKT3 are described in SEQ ID NO:1, SEQ ID NO:2, and SEQ ID NO:3 for the heavy chain variable domain and in SEQ ID NO:4, SEQ ID NO:5, and SEQ ID NO:6 for the light chain variable domain (see FIG 2A and FIG 2B for the IMGT nomenclature). The framework for humanization of OKT3 is based on published sequences in Adair et al. (1994) Hum Antibodies Hybridoma 5, 41-47. The CDRs (according to IMGT nomenclature) of anti-CD28 antibody clone 15E8 are described in SEQ ID NO:7, SEQ ID NO:8, and SEQ ID NO:9 for the heavy chain variable domain and in SEQ ID NO: 10, SEQ ID NO:11, and SEQ ID NO:12 for the light chain variable domain. The framework for humanization of 15E8 is based on human germline framework sequences.

Beside the antibodies or fragments thereof disclosed herein, the present disclosure also comprises isolated polynucleotides encoding for said antibodies or fragments thereof, expression vectors comprising said polynucleotides, an expression system comprising said polynucleotides and host cells expressing said antibodies or fragment thereof, host cells comprising said polynucleotides, the use of said antibodies or fragments thereof for stimulation of T cells, and use of said antibodies or fragments thereof for reversible tagging of cells.

### Brief description of the drawings

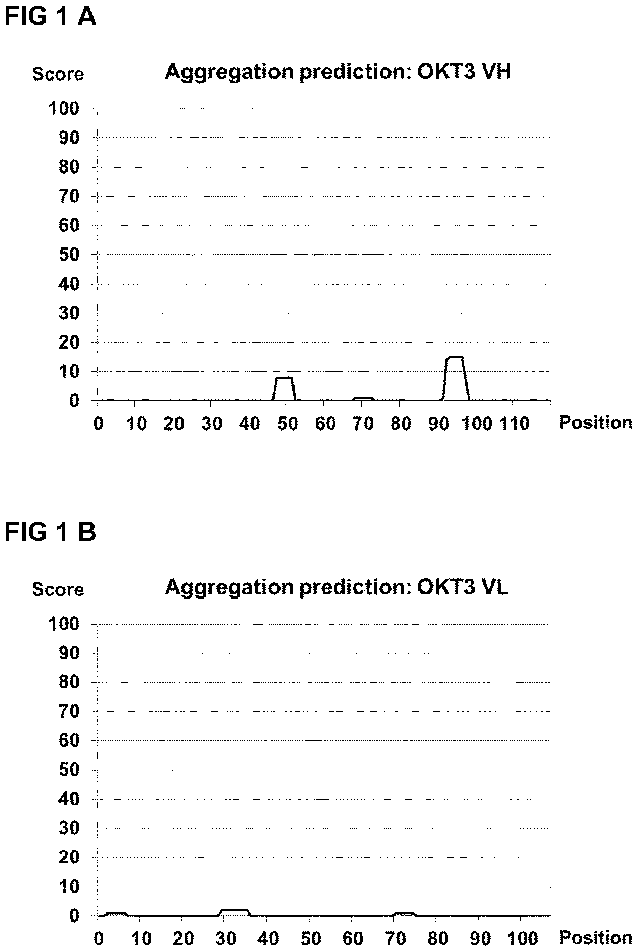

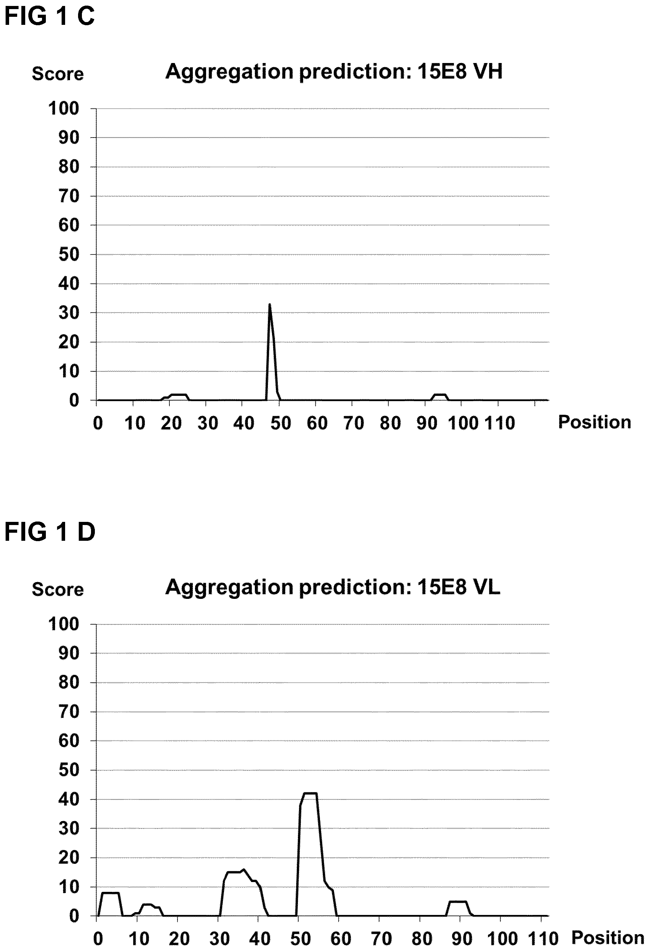

FIG 1: *In silico* aggregation prediction of parental murine anti-CD3 antibody clone OKT3 heavy chain (VH, FIG 1A) and light chain (VL, FIG 1B) variable domains and anti-CD28 antibody clone 15E8 heavy chain (VH, FIG 1C) and light chain (VL, FIG 1D) variable domains. For each amino acid residue, given here by a position number, a numerical score was calculated using standard software known in the art which predicts the tendency of protein aggregation. Higher scores stand for an increased aggregation tendency. The minimum score is 0 (not aggregation-prone) and the maximum score is 100 (highly aggregation-prone).
FIG 2: Definition of framework regions FR1, FR2, FR3, FR4 and CDR regions CDR1, CDR2, and CDR3 using heavy (FIG 2A) and light (FIG 2B) chain variable domains of parental murine 15E8 antibody specific for CD28 antigen as an example. Numbering is according to IMGT.

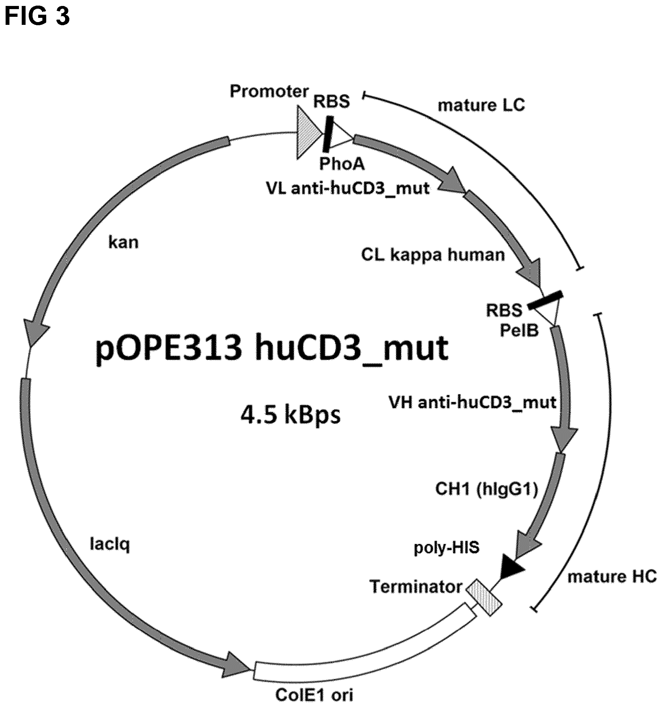

FIG 3: Plasmid map of the prokaryotic vector for the expression of Fab fragments specific for CD3 antigen and specific for CD28 antigen in *E.coli.* LC, light chain; HC heavy chain; VL, light chain variable domain; VH, heavy chain variable domain; CL light chain constant domain; CH1 (hIgG1), CH1 domain of human IgG1; RBS, ribosome binding site; poly-HIS, poly-histidine sequence; kan, kanamycin resistance gene; lacIq, lac repressor gene; ori, origin of replication; PhoA, signal peptide PhoA; PelB, signal peptide PelB; kBps, kilo base pairs.

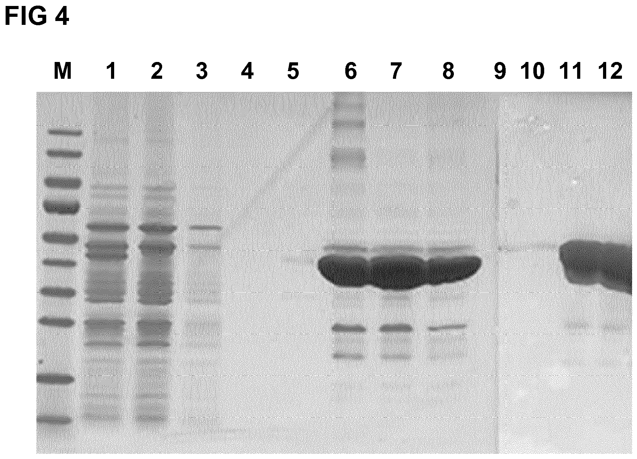

FIG 4: Coomassie-stained SDS gel with samples of different steps of the purification of Fab variant "huCD3_mut" by metal chelate affinity and ion exchange chromatography. M, molecular weight marker with sizes of 170, 130, 100, 70, 55, 40, 35, 25, 15, and 10 kDa from top to bottom. Lane 1: periplasmic extract; lane 2: metal chelate flow-through fraction; lanes 3-4: wash fractions; lanes 5-8: eluate fractions; lane 9: ion exchange flow-through fraction; lane 10: wash fraction; lanes 11-12: eluate fractions.

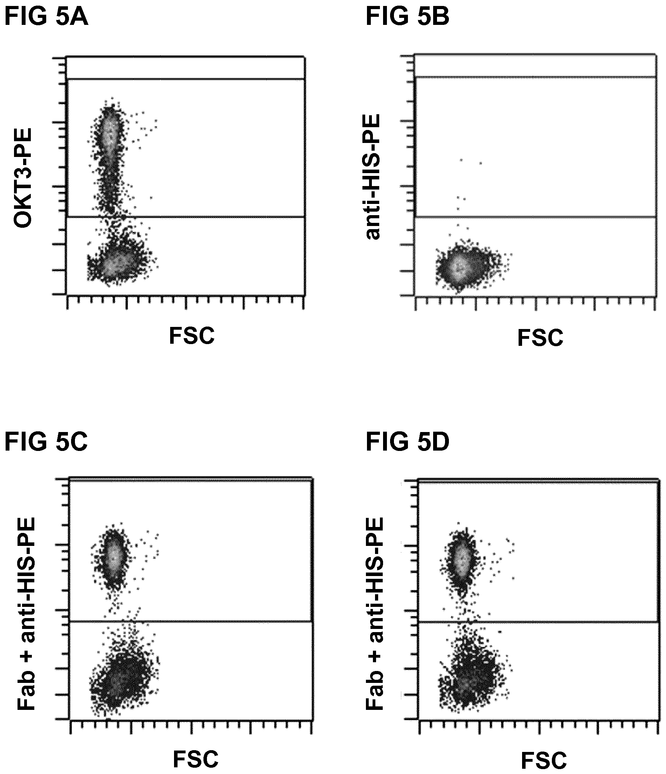

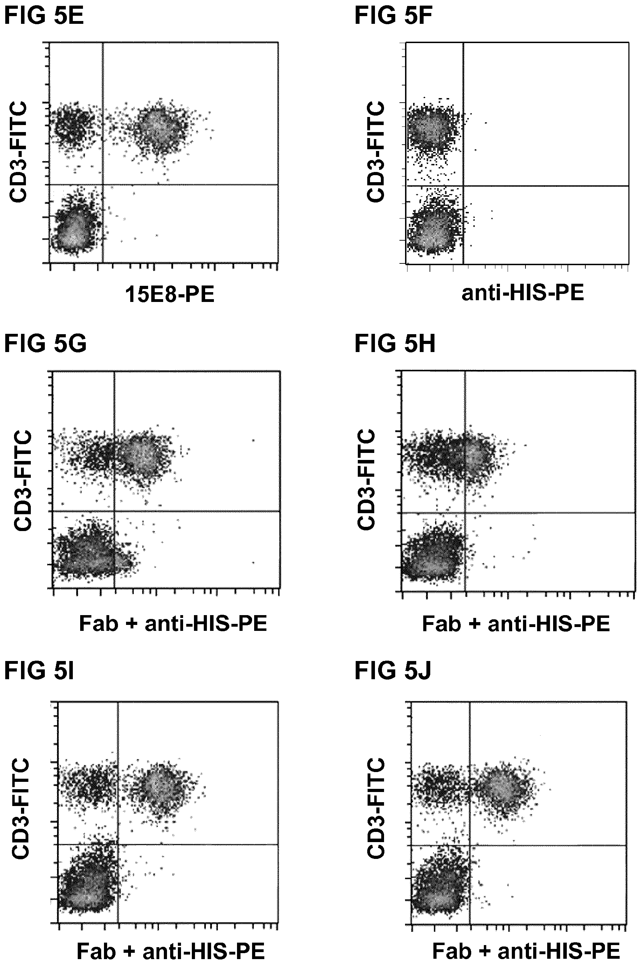

FIG 5: Flow cytometry analysis of PBMC stained either using direct antibody conjugates or indirectly using Fabs in combination with an anti-polyhistidine antibody. 5A-5D: dot plots of anti-CD3 stainings; 5A: positive control, OKT3-PE conjugate; 5B: negative control, anti-polyhistidine-PE conjugate; 5C: chCD3_mut Fab; 5D: huCD3_mut Fab. 5E-5J: dot plots of anti-CD28 stainings; 5E: positive control, 15E8-PE conjugate; 5F: negative control, anti-polyhistidine-PE conjugate; 5G: huCD28_v1 Fab; 5H: huCD28_v2 Fab; 5I: huCD28_v3 Fab; 5J: huCD28_v4 Fab. FSC: forward scatter. PE: phycoerythrin. FITC: fluorescein isothiocyanate. anti-HIS-PE: anti-polyhistidine-phycoerythrin. The range of the x-axis is from 0 to 1000, and of the y-axis from 1E-01 to 1E+03.

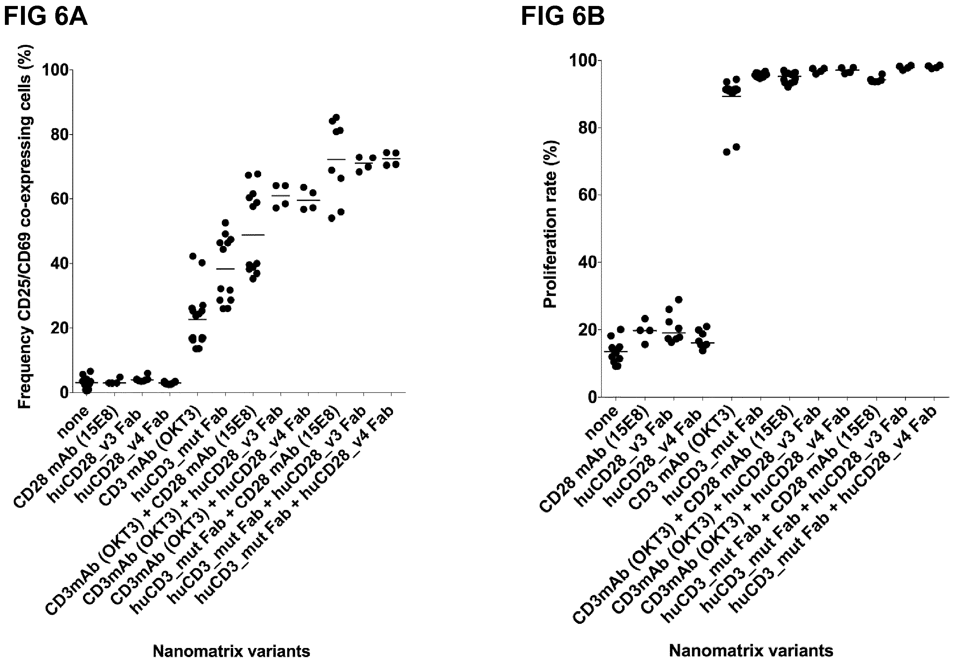

FIG 6: Stimulation of T cells by different combinations of nanomatrices comprising antibodies or fragments thereof specific for CD3 and CD28. 6A: frequency of T cells (%) expressing both early activation markers CD25 and CD69 after 48 hours of stimulation. 6B: proliferation rate (%) of T cells after one week of stimulation. Each dot represents the sample of one donor. Horizontal lines represent the corresponding median value. "mAb" denotes the full-length murine parental antibody (OKT3 for CD3 and 15E8 for CD28).

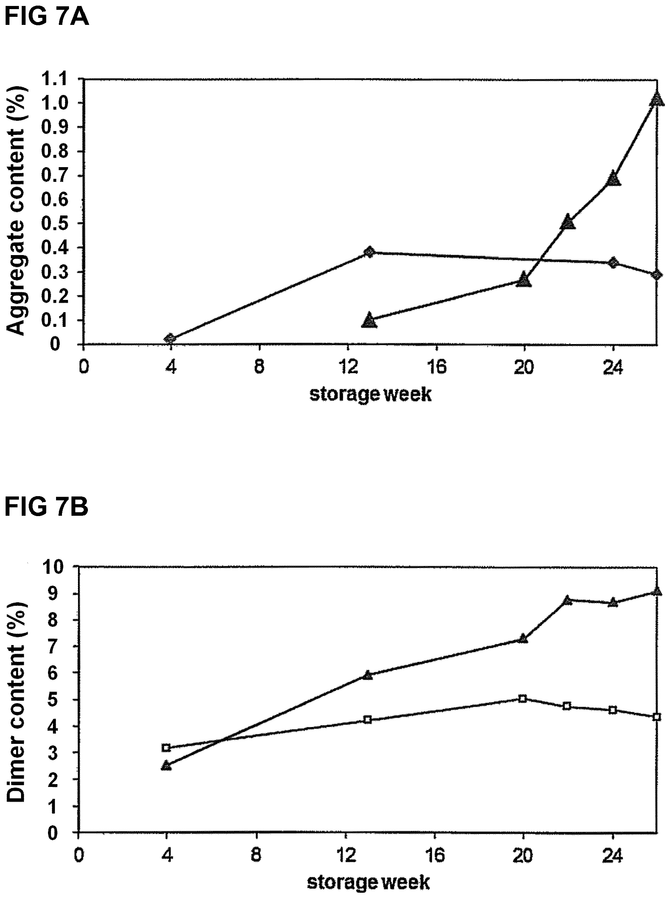

FIG 7: SEC-HPLC results of purified parental murine antibody clones OKT3 (anti-CD3) and 15E8 (anti-CD28) after storage at 25 °C for 26 weeks. 7A: amount (%) of aggregated antibody, triangles: OKT3, diamonds: 15E8; 7B: amount (%) of dimeric antibody, closed triangles: OKT3, open squares: 15E8.

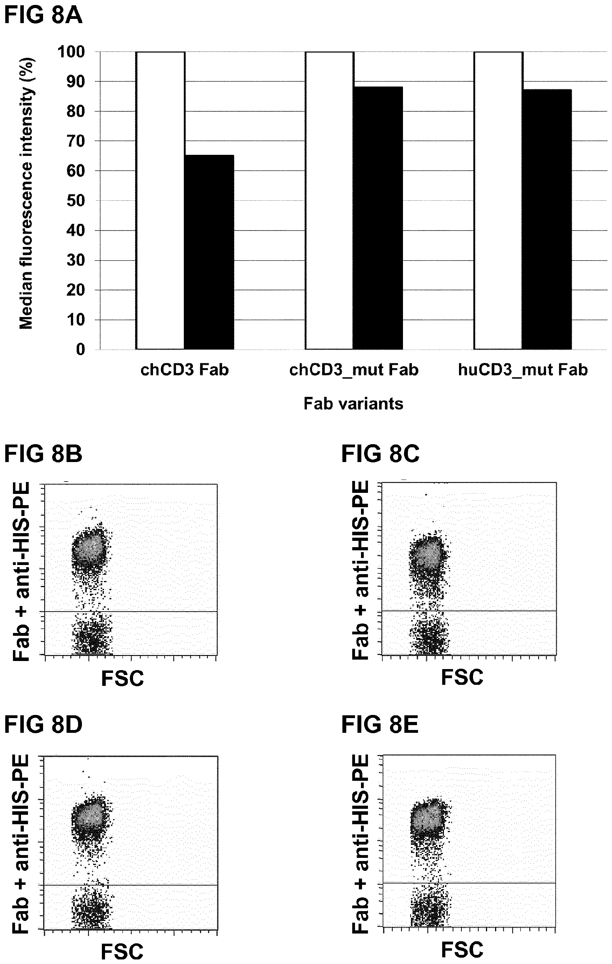

FIG 8: Staining of PBMC using thermally stressed anti-CD3 Fabs. 8A: Median fluorescence intensity (%) of stained cells; open columns: using non-incubated Fabs, each set to 100% (reference); filled columns: using Fabs after incubation at 37 °C for one week. 8B-E: Flow cytometry analysis; dot plots of 8B: non-incubated chCD3 Fab, 8C: incubated (stressed) chCD3 Fab, 8D: non-incubated huCD3_mut Fab, 8E: incubated (stressed) huCD3_mut Fab. FSC: forward scatter. anti-HIS-PE: anti-polyhistidine-phycoerythrin. The range of the x-axis is from 0 to 1000, and of the y-axis from 1E-01 to 1E+03.

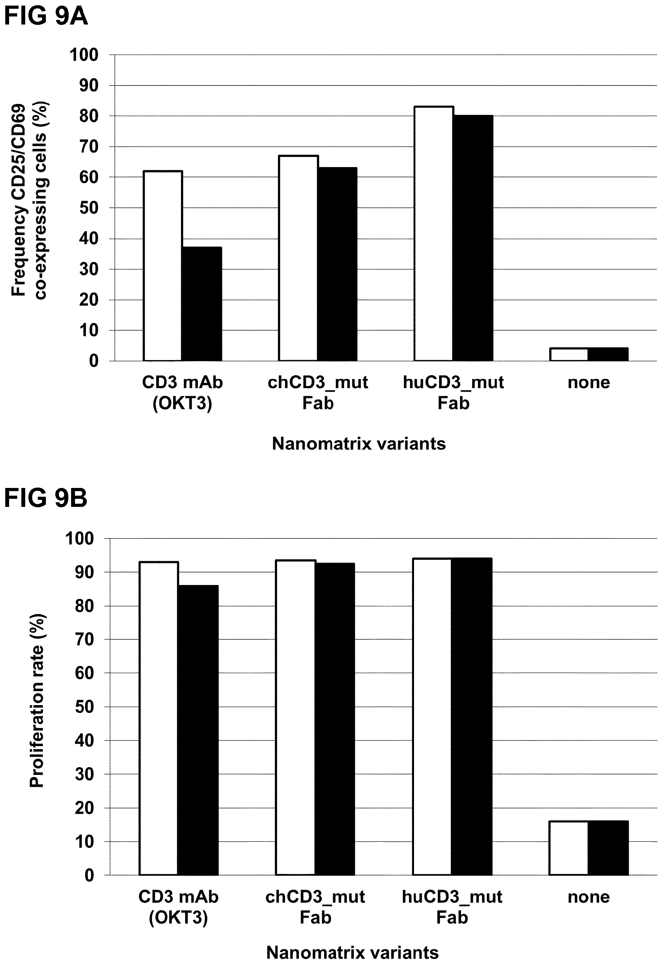

FIG 9: Stimulation of T cells using thermally stressed nanomatrices comprising antibodies or fragments thereof. Open columns: using non-incubated nanomatrices (reference); filled columns: using nanomatrices after incubation at 37 °C for one week. 9A: frequency of T cells (%) expressing both early activation markers CD25 and CD69 after 48 hours of stimulation. 9B: proliferation rate (%) of T cells after one week of stimulation. "mAb" denotes the full-length murine parental antibody OKT3 (for CD3).

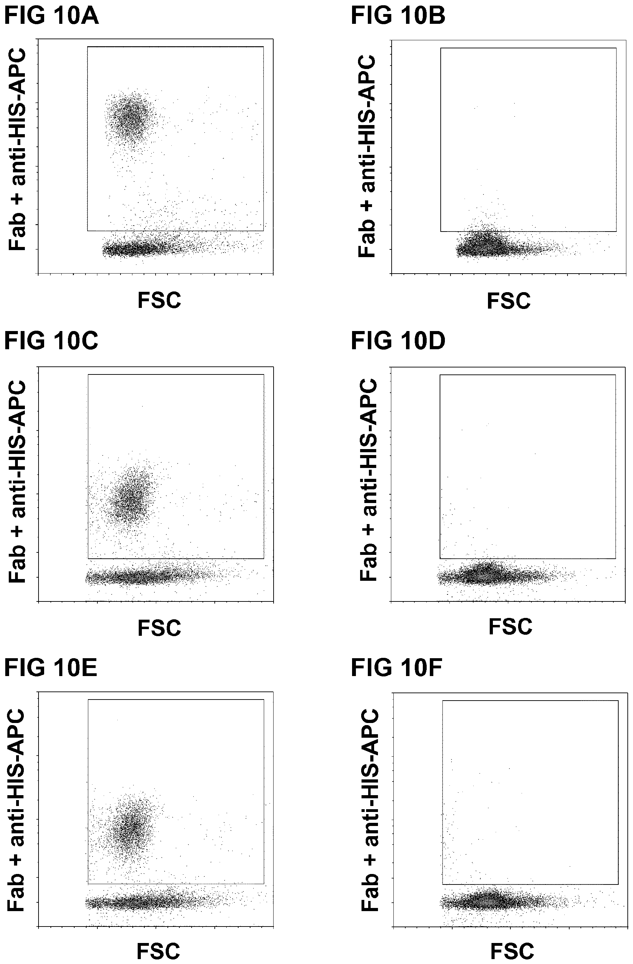

FIG 10: Reversible tagging of PBMC using Fabs specific for CD3 and CD28 in combination with an anti-polyhistidine-fluorochrome conjugate, analyzed by flow cytometry. A, B: dot plots for CD3; D-F: dot plots for CD28. A, C, E: tagged cells before washing; B, D, F: removed tagging after washing of the cells. anti-HIS-APC: anti-polyhistidine-allophycocyanin. FSC: forward scatter. The range of the x-axis is from 0 to 1000, and of the y-axis from 1E-01 to 1E+03.

### Detailed description of the invention

In a an aspect the present disclosure provides a humanized antibody or fragment thereof specific for the antigen CD3, wherein said antibody or fragment thereof comprises a heavy chain variable domain comprising a CDR1 region of SEQ ID NO:1, a CDR2 region of SEQ ID NO:2, and a CDR3 region of SEQ ID NO:3, and a light chain variable domain comprising a CDR1 region of SEQ ID NO:4, a CDR2 region of SEQ ID NO:5, and a CDR3 region of SEQ ID NO:6. Different embodiments of said humanized antibody or fragment thereof are disclosed herein.

Said CDRs may be embedded into a framework sequence comprising the regions FR1, FR2, FR3, and FR4. The order of sequence may be FR1 - CDR1 - FR2 - CDR2 - FR3 - CDR3 - FR4. The heavy chain variable domain of said humanized antibody or fragment thereof may comprise framework regions FR1, FR2, FR3, and FR4, wherein said framework regions FR1, FR2, FR3, and FR4 are amino acid sequences having an identity of at least 70%, more preferentially at least 80%, most preferentially at least 90% to the framework regions FR1, FR2, FR3, and FR4 of SEQ ID NO:16. The light chain variable domain of said humanized antibody or fragment thereof may comprise framework regions FR1, FR2, FR3, and FR4, wherein said framework regions FR1, FR2, FR3, and FR4 are amino acid sequences having an identity of at least 70%, more preferentially at least 80%, most preferentially at least 90% to the framework regions FR1, FR2, FR3, and FR4 of SEQ ID NO:17.

The framework regions of said heavy chain variable domain and said light chain variable domain may comprise amino acid substitutions (backmutations) to increase the affinity of the antibody or fragment thereof compared to the humanized variant without backmutation. Furthermore, backmutations may affect the amount of antibody which can be manufactured by stabilizing or destabilizing the protein structure. Preferentially, said heavy chain variable domain comprises the amino acid sequence of SEQ ID NO:16 and said light chain variable domain comprises the amino acid sequence of SEQ ID NO:17. Preferentially, the antibodies or fragments thereof bind specifically to CD3 antigen with an affinity of at least 20%, 30%, 50%, 70%, 90%, 100%, or more than 100% for the CD3 antigen when compared to the parental murine anti-CD3 antibody clone OKT3 or fragments thereof.

In an aspect the invention provides an *in-vitro* method for polyclonal stimulation of T cells comprising contacting a population of T cells with a humanized anti-CD3 antibody fragment, wherein said humanized anti-CD3 antibody fragment comprises a heavy chain variable domain comprising the sequence of SEQ ID NO:16, and a light chain variable domain comprising the sequence of SEQ ID NO:17, and wherein said antibody or fragment thereof is linked to particles.

Said particles may be beads with a solid phase surface ranging in size between 500 nm to 10 µm. Alternatively, said particles may be a nanomatrix, the nanomatrix comprising a) a matrix of mobile polymer chains, and b) attached to said matrix of mobile polymer chains said antibody or fragment thereof, wherein the nanomatrix is 1 to 500 nm in size.

Said anti-CD3 antibody fragment may be used in combination with an anti-CD28 antibody or fragment thereof. Said anti-CD28 antibody or fragment thereof may be humanized. Said anti-CD28 antibody or fragment thereof may be humanized clone 15E8 as disclosed herein. Said anti-CD28 antibody or fragment thereof may comprise a humanized heavy chain variable domain comprising a CDR1 region of SEQ ID NO:7, a CDR2 region of SEQ ID NO:8, and a CDR3 region of SEQ ID NO:9, and a humanized light chain variable domain comprising a CDR1 region of SEQ ID NO:10, a CDR2 region of SEQ ID NO:11, and a CDR3 region of SEQ ID NO: 12. Said anti-CD28 antibody or fragment thereof may be linked to particles.

The CDRs of said anti-CD28 antibody or fragment thereof may be embedded into a framework sequence comprising the regions FR1, FR2, FR3, and FR4. The order of sequence may be FR1 - CDR1 - FR2 - CDR2 - FR3 - CDR3 - FR4. The humanized heavy chain variable domain of said humanized antibody or fragment thereof may comprise framework regions FR1, FR2, FR3, and FR4, wherein said framework regions FR1, FR2, FR3, and FR4 are amino acid sequences having an identity of at least 70%, more preferentially at least 80%, most preferentially at least 90% to the framework regions FR1, FR2, FR3, and FR4 of either SEQ ID NO:26 or SEQ ID NO:24.

The humanized light chain variable domain of said humanized anti-CD28 antibody or fragment thereof may comprise framework regions FR1, FR2, FR3, and FR4, wherein said framework regions FR1, FR2, FR3, and FR4 are amino acid sequences having an identity of at least 70%, more preferentially at least 80%, most preferentially at least 90% to the framework regions FR1, FR2, FR3, and FR4 of SEQ ID NO:25.

The framework regions of said humanized anti-CD28 heavy chain variable domain and said humanized anti-CD28 light chain variable domain may comprise amino acid substitutions (backmutations) to increase the affinity of the antibody or fragment thereof compared to the humanized variant without backmutation. Furthermore, backmutations may affect the amount of antibody which can be manufactured by stabilizing or destabilizing the protein structure. Preferentially, the light chain variable domain comprises the amino acid substitutions D74A (aspartate-74 to alanine) and S93P (serine-93 to proline, according to IMGT nomenclature) as backmutations. Preferentially, said humanized heavy chain variable domain comprises the amino acid sequence of either SEQ ID NO:26 or SEQ ID NO:24, and said humanized light chain variable domain comprises the amino acid sequence of SEQ ID NO:25. Preferentially, the antibodies or fragments thereof bind specifically to CD28 with an affinity of at least 20%, 30%, 50%, 70%, 90%, 100%, or more than 100% for the CD28 antigen when compared to the parental murine anti-CD28 antibody clone 15E8 or fragments thereof.

As a component of a nanomatrix, the antibody fragments specific for CD3 and CD28, respectively, may be linked to the same or to a different matrix of mobile polymer chains: they may be localized on the same matrix (for example a matrix bearing both antibodies or fragments thereof specific for CD3 and CD28) or on separated matrices (for example one matrix bearing an antibody or fragment thereof specific for CD3 and another matrix bearing an antibody or fragment thereof specific for CD28). When localized on the same matrix, the molar ratio of the antibodies or fragments thereof specific for CD3 and CD28 may vary, for example 100:1, 10:1, 3:1, 1:1, 1:3, 1:10, and 1:100 (anti-CD3:anti-CD28) ratios may be useful. Said method of polyclonal stimulation of T cells may be performed in a closed and sterile cell culture system (closed cell sample processing system).

In another aspect the present disclosure provides a cell composition obtained by the method of the present invention for therapeutic applications.

In a further aspect the present disclosure provides the use of an anti-CD3 antibody or fragment thereof for reversible tagging of cells, wherein said anti-CD3 antibody or fragment thereof comprises a humanized heavy chain variable domain comprising a CDR1 region of SEQ ID NO:1, a CDR2 region of SEQ ID NO:2, and a CDR3 region of SEQ ID NO:3, and a humanized light chain variable domain comprising a CDR1 region of SEQ ID NO:4, a CDR2 region of SEQ ID NO:5, and a CDR3 region of SEQ ID NO:6, and wherein said antibody or fragment thereof is linked to a tag.

The present disclosure also provides the use of said anti-CD3 antibody or fragment thereof in combination with an anti-CD28 antibody or fragment thereof for reversible tagging of cells, wherein said anti-CD28 antibody or fragment thereof comprises a humanized heavy chain variable domain comprising a CDR1 region of SEQ ID NO:7, a CDR2 region of SEQ ID NO:8, and a CDR3 region of SEQ ID NO:9, and a humanized light chain variable domain comprising a CDR1 region of SEQ ID NO: 10, a CDR2 region of SEQ ID NO:11, and a CDR3 region of SEQ ID NO:12, and wherein said antibody or fragment thereof is linked to a tag.

Said tag may be particles (e.g. a bead), labels (e.g. a fluorescent dye, an isotopic label, or a magnetic entity), molecules (e.g. a nucleic acid, a protein, or a peptide), or surfaces (e.g. a culture dish or a microtiter plate). The tagging may be dissolved by allowing a monovalent binding of said antibodies or fragments thereof to their antigen, resulting in a dissociation of the antibody-antigen complex due to the kinetic properties of the antibodies or fragments thereof.

The antibodies or fragments thereof specific for CD3 or CD28, respectively, may be fusion proteins. Examples for fusions comprise poly-histidine sequences, biotinylation sequences, biotin-mimicking peptides, fluorescent proteins, linker sequences such as poly-glycine-serine, and chimeric antigen receptors.

The antibodies or fragments thereof specific for CD3 or CD28, respectively, may contain amino acid substitutions, insertions and deletions. The antibodies or fragments thereof specific for CD3 or CD28, respectively, may be chemically modified. Examples comprise conjugation to fluorescent dyes, to proteins, and to magnetic particles, and chemical modifications known in the art such as biotinylation.

In one aspect the present disclosure provides isolated polynucleotides encoding for humanized antibodies or fragments thereof specific for CD3 antigen as disclosed herein.

In another aspect the present disclosure provides an expression system comprising the polynucleotides encoding an antibody or fragment thereof specific for CD3 antigen as disclosed herein, and a host cell expressing said antibody or fragment thereof.

In a further aspect the present disclosure provides a host cell comprising the polynucleotides encoding the antibodies or fragments thereof as disclosed herein.

### Definitions

Unless defined otherwise, technical and scientific terms used herein have the same meaning as commonly understood by one of ordinary skill in the art to which this invention belongs.

The term "antibody" as used herein is used in the broadest sense to cover the various forms of antibody structures including but not being limited to monoclonal and polyclonal antibodies (including full length antibodies), multispecific antibodies (e.g. bispecific antibodies), antibody fragments, immunoadhesins and antibody-immunoadhesin chimeras, that specifically recognize (i.e. bind) a target antigen. The antibody according to the invention is preferably a humanized antibody, chimeric antibody, or further genetically engineered antibody as long as the characteristic properties according to the invention are retained. "Antibody fragments" comprise a portion of a full length antibody, preferably the variable domain thereof, or at least the antigen binding site thereof. Examples of antibody fragments include Fab (fragment antigen binding), scFv (single chain fragment variable), single domain antibodies, diabodies, dsFv, Fab', diabodies, single-chain antibody molecules, and multispecific antibodies formed from antibody fragments. scFv antibodies are, e.g., described in Ahmad et al. (2012) Clin Dev Immunol. 2012, 980250. The term "humanized antibody" refers to antibodies in which the framework and/or CDRs have been modified to comprise the CDR of an immunoglobulin of different species as compared to that of the parent immunoglobulin. In a preferred embodiment, a rodent (e.g., mouse) CDR is grafted into the framework region of a human antibody to prepare the "humanized antibody" (an example is given in Riechmann et al. (1988) Nature 332, 323-327). A humanized antibody binds to the same or similar antigen as the donor antibody that provides the CDRs. The acceptor framework of a humanized immunoglobulin may have a limited number of substitutions by amino acids taken from the donor framework. Humanized molecules can have additional conservative amino acid substitutions which have substantially no effect on antigen binding or other immunoglobulin functions. Conservative substitutions generally maintain (a) the structure of the polypeptide backbone in the area of the substitution, for example, as a sheet or helical conformation, (b) the charge or hydrophobicity of the molecule at the target site, and/or (c) the bulk of the side chain.

As used herein, the terms "parent" and "parental" with regard to an antibody sequence refer to the antibody sequence that is to be humanized. A parental antibody therefore is the nonhuman antibody clone to be humanized, usually of a rodent origin. The murine antibody clone OKT3 specific for CD3 antigen of the present invention is an example of a parental antibody. As used herein, the term "antigen" is intended to include substances that evoke the production of one or more antibodies. Each antibody binds to a specific antigen by way of an interaction similar to the fit between a lock and a key. The substance may be from the external environment or formed within the body. The term "antigen" comprises, but is not limited to, proteins, peptides, polypeptides, oligopeptides, lipids, carbohydrates, and combinations thereof, for example a glycosylated protein or a glycolipid. An antigen may be on the cell surface or inside the cell. Preferentially, an antigen is on the cell surface of a cell. Preferentially, the CD3 antigen is a mammalian CD3 antigen. Most preferred, the CD3 antigen is a human CD3 antigen. Preferentially, the CD28 antigen is a mammalian CD28 antigen. Most preferred, the CD28 antigen is a human CD28 antigen.

The terms "specifically binds to" or "specific for" with respect to an antigen-binding domain of an antibody or fragment thereof refer to an antigen-binding domain which recognizes and binds to a specific antigen, i.e. CD3 in the case of an anti-CD3 antibody or fragment thereof, but does not substantially recognize or bind other antigens in a sample. An antigen-binding domain that binds specifically to an antigen from one species may bind also to that antigen from another species. This cross-species reactivity is not contrary to the definition of that antigen-binding domain as specific. An antigen-binding domain that specifically binds to an antigen may bind also to different allelic forms of the antigen (allelic variants, splice variants, isoforms etc.). This cross reactivity is not contrary to the definition of that antigen-binding domain as specific.

The term "domain" refers to a protein structure which retains its tertiary structure independently of the remainder of the protein. In some cases, domains have discrete functional properties and can be added, removed or transferred to another protein without a loss of function.

The term "affinity" refers to the strength of the sum total of non-covalent interactions between a single binding site of a molecule (e.g., binding arm of an antibody) and its binding partner (e.g., an antigen). Unless indicated otherwise, as used herein, "binding affinity" refers to intrinsic binding affinity which reflects an 1:1 interaction between members of a binding pair (e.g., antigen-binding protein and its antigen). The affinity of a molecule X for its partner Y can generally be represented by the equilibrium dissociation constant (K_{D}). Affinity can be measured by common methods known in the art (e.g., Biacore^{™} measurement).

The "variable domain" or "variable region" (variable domain of a light chain (VL), variable domain of a heavy chain (VH)) as used herein denotes each of the pair of light and heavy chain domains which are involved directly in binding of the antibody to the antigen. The term "variable domain" refers to both variable domains of immunoglobulin light chains of the kappa type and variable domains of immunoglobulin light chains of the lambda type.

The "constant domain" or "constant region" (constant domain of a light chain (CL), constant domain of a heavy chain (CH)) as used herein denotes the domains that comprise the CL domain (in the case of the light chain) and the CH1, Hinge, CH2, CH3 and/or CH4 domains (in the case of the heavy chain) of an antibody. The constant region of the heavy chain differs in antibodies of different isotypes. In recombinant antibodies or fragments thereof, CH1, Hinge, CH2, CH3 and CH4 domains and fragments thereof of different isotypes may be combined. CH1, Hinge, CH2, CH3 and CH4 domains may also be chosen from different species. In a humanized Fab fragment, the constant domain is preferentially a human constant domain, more preferentially human CL and human IgG1 CH1.

The variable light and heavy chain domains have the same general structure and each domain comprises four framework regions (FR) whose sequences are widely conserved, connected by three "hypervariable regions" (or complementary determining regions, CDRs). The light and heavy chain variable domains of an antibody comprise, from N- to C-terminus, the domains FR1, CDR1, FR2, CDR2, FR3, CDR3, and FR4. The CDRs in each chain are held in their three-dimensional structure by the framework regions and form together with the CDRs from the other chain the antigen binding site. The assignment of CDR and FR regions to the antibody sequence as used herein is determined according to the IMGT definition (http://www.imgt.org), also referred to as FR-IMGT and CDR-IMGT. There are other definitions of FR and CDR regions according to, for example, Kabat and Chothia, which may result in slightly different assignments of amino acid positions at the conjunctions of FR and CDR regions and therefore in slightly different CDR lengths. The antibody according to the invention may comprise an Fc part from human origin which is selected from the subclasses IgG1, IgG2, IgG3, and IgG4. Preferentially the Fc part from human origin is of the subclass IgG1 or IgG4. The Fc part may contain amino acid substitutions, insertions and deletions. For example, amino acid residues involved in Fc gamma receptor binding may be substituted with other amino acid residues to increase, minimize, or abolish this interaction.

The antibody according to the invention can be a fragment thereof. The antibody fragment may contain amino acid substitutions, insertions and deletions. For example, a poly-histidine sequence may be inserted or added to any Fab fragment chain which allows for purification using affinity chromatography and/or immunological detection using anti-histidine antibodies. The antibody according to the invention is preferably characterized in that the constant domains are of human origin. Such constant domains are well known in the art and described, e.g. by Kabat.

As used herein, the term "CD3 antigen" refers to the CD3 protein, also known as T-cell surface glycoprotein CD3, and the term "CD28 antigen" refers to the CD28 protein, also known as T-cell-specific surface glycoprotein CD28.

As used herein, the term "IMGT nomenclature" describes the numbering scheme of antibody sequences according to IMGT (ImMunoGeneTics Information system^{®}, www.imgt.org; Giudicelli et al. (2005) Nucleic Acids Res. 33, D256 - D261). FIG 2A and 2B exemplarily show the IMGT nomenclature for the heavy and light chain variable domains of an anti-CD28 antibody.

As used herein, the term "IGKV" is used as abbreviation for immunoglobulin kappa variable region genes. The term "IGHV" is used as abbreviation for immunoglobulin heavy chain variable region genes.

As used herein, the term "CDR grafting" refers to a method of taking CDR regions of one antibody or fragment thereof and transferring these into the framework of a second antibody or fragment thereof wherein the receiving framework (acceptor framework) is not identical to the framework of the first antibody (donor framework).

As used herein, the terms "framework" and "framework regions" refer to the amino acid sequence of an antibody or fragment thereof comprising domains FR1, FR2, FR3, and FR4 of both heavy and light chain variable regions, i.e the variable domains without the CDR regions. Accordingly, the terms "germline framework" and "germline framework regions" refer to the variable antibody domains without the CDR regions encoded by human germline antibody genes.

As used herein, the term "identity" (of proteins and polypeptides) with respect to amino acid sequences is used for a comparison of proteins chains. Calculations of "sequence identity" between two sequences are performed as follows. The sequences are aligned for optimal comparison purposes (e.g., gaps can be introduced in one or both of a first and a second amino acid sequence for optimal alignment and non-homologous sequences can be disregarded for comparison purposes). The optimal alignment is determined as the best score using the "ssearch36" program in the FASTA36 software package (http://faculty.virginia.edu/wrpearson/fasta/) with a Blossum 50 scoring matrix with a gapopen penalty of -10, and a gap-extension penalty of -2. The amino acid residues at corresponding amino acid positions are then compared. When a position in the first sequence is occupied by the same amino acid residue at the corresponding position in the second sequence, then the molecules are identical at that position. The percent identity between the two sequences is a function of the number of identical positions shared by the sequences.

As used herein, the term "backmutation" refers to an amino acid substitution in the framework region of a humanized antibody sequence. This is usually done to increase the affinity of the antibody towards its antigen after humanization. Candidate residues for backmutation comprise, for example, positions with different amino acids in the humanized antibody after CDR grafting and the parental non-humanized antibody, and positions with different amino acids in the humanized antibody after CDR grafting and an antibody consensus sequence. Usually candidate residues are at sites of the framework regions that are important for the binding properties of the antibody and/or that determine the main chain conformations of the antigen binding loops.

Unlike in CDRs, more substantial changes in structure framework regions (FRs) can be made without adversely affecting the binding properties of an antibody. Changes to FRs include, but are not limited to, humanizing a nonhuman-derived framework or engineering certain framework residues that are important for antigen contact or for stabilizing the binding site, e.g., changing the class or subclass of the constant region, changing specific amino acid residues which might alter an effector function such as Fc receptor binding (Lund et al. (1991) J. Immunol. 147, 2657-2662; Morgan et al. (1995) Immunology 86, 319-324), or changing the species from which the constant region is derived.

The antibodies of the invention are typically recombinantly expressed polypeptides. The antibodies may be chimeric polypeptides, wherein the term "chimeric polypeptide" refers to an artificial (non-naturally occurring) polypeptide which is created by juxtaposition of two or more peptide fragments which do not otherwise occur contiguously. In the case of a Fab molecule, the term "chimeric Fab" refers to the combination of variable and constant regions of different origin, for example the combination of variable regions of murine origin and constant regions of human origin. The Fab variant "chCD3_mut Fab" is an example for a chimeric Fab.

As used herein, the terms "positive selection of cells" and "enrichment of cells" as used herein have an interchangeable meaning and refer to the isolation of a subpopulation of cells from a cell population including tissue. Methods suitable for the positive selection of cells comprise centrifugation, filtration, magnetic cell sorting, and fluorescent cell sorting.

As used herein, the term "depletion of cells" as used herein refers to a process of negative selection that separates desired cells from undesired cells. Samples of cells as used herein may comprise cells of blood origin such as PBMC, of cell culture origin, or of tissue origin such as brain or bone which may be dissociated before use. Methods suitable for the depletion of cells comprise centrifugation, filtration, magnetic cell sorting, and fluorescent cell sorting.

The terms "CD3-expressing" and "CD3-positive" (CD3+) cells as used herein have an interchangeable meaning and describe cells which express the CD3 antigen. Accordingly, the terms "CD3 non-expressing" and "CD3-negative" (CD3-) cells as used herein have an interchangeable meaning and describe cells which do not express the CD3 antigen in a detectable manner.

The amino acid sequences of a CDR1 region, a CDR2 region and a CDR3 region of a humanized heavy chain variable domain, and the amino acid sequences of a CDR1 region, a CDR2 region and a CDR3 of a humanized light chain variable domain are given in SEQ ID NO:1, SEQ ID NO:2, SEQ ID NO:3, SEQ ID NO:4, SEQ ID NO:5 and SEQ ID NO:6, respectively. The amino acid sequences of a heavy chain variable domain and a light chain variable domain are given in SEQ ID NO:13 and SEQ ID NO:14, respectively.

The antibodies or fragments thereof according to the invention are preferentially produced by recombinant means. Such methods are widely known in the state of the art and comprise protein expression in prokaryotic and eukaryotic cells, preferentially in non-mammalian cells, with subsequent isolation of the antibody polypeptide (full antibody or fragment thereof). The purification of the antibody polypeptide may be performed to a pharmaceutically acceptable purity. For the protein expression nucleic acids encoding light and heavy chains or fragments thereof may be inserted into expression vectors or viral transduction vectors by standard methods. Expression is performed in appropriate prokaryotic or eukaryotic host cells, such as CHO cells, NS0 cells, SP2/0 cells, HEK293 cells, COS cells, yeast, Bacillus or *E.coli* cells, and the antibody is recovered from the cells (from the supernatant or after full or partial cells lysis). Recombinant production of antibodies is well-known in the state of the art.

The term "therapeutic effective amount" means an amount which provides a therapeutic benefit.

The term "isolated" means altered or removed from the natural state. For example an isolated population of cells means an enrichment of such cells and separation from other cells which are normally associated in their naturally occurring state with said isolated cells. An isolated population of cells means a population of substantially purified cells which are a homogenous population of cells.

The terms "T cell stimulation" and "T cell activation" as used herein have an interchangeable meaning and refer to a cellular response which is characterized by T cell proliferation and expression of early T cell activation markers, especially CD25 and CD69.

The term "CDR-mutated variant" as used herein refers to a variant of an antibody or fragments thereof comprising at least one amino acid substitution, insertion, or deletion within one of the six CDR regions. The amino acid substitution cysteine to serine at position 114 (IMGT nomenclature) in the CDR3 of the heavy chain variable domain of anti-CD3 clone OKT3 is an example for a CDR-mutated variant. Accordingly, the term "non-CDR-mutated variant" as used herein refers to the variant of an antibody or fragments thereof comprising no amino acid substitution, insertion, or deletion within one of the six CDR regions (i.e. the original CDR regions are used).

The term "tag" as used herein refers to the coupling of the antigen-binding molecule, e.g. an antibody or fragment thereof, to other molecules, e.g. particles, fluorophores, haptens like biotin, or larger surfaces such as culture dishes and microtiter plates. In some cases the coupling results in direct immobilization of the antigen-binding molecule, e.g. if the antigen binding molecule is coupled to a larger surface of a culture dish. In other cases this coupling results in indirect immobilization, e.g. an antigen-binding molecule coupled directly or indirectly (via e.g. biotin) to a magnetic bead is immobilized if said bead is retained in a magnetic field. In further cases the coupling of the antigen-binding molecule to other molecules does not result in a direct or indirect immobilization but allows for enrichment, separation, isolation, and detection of cells according to the present invention, e.g. if the antigen-binding molecule is coupled to a fluorophore which then allows discrimination of stronger labeled cells, weaker labeled cells, and non-labeled cells, e.g. via flow cytometry methods, like FACSorting, or fluorescence microscopy.

The term "particle" as used herein refers to a solid phase such as colloidal particles, microspheres, nanoparticles, or beads. Methods for generation of such particles are well known in the field of the art. The particles may be magnetic particles. The particles may be in a solution or suspension or they may be in a lyophilized state prior to use in the present invention. The lyophilized particle is then reconstituted in convenient buffer before contacting the sample to be processed regarding the present invention.

The terms "matrix of mobile polymer chains" and "mobile matrix" as used herein have an interchangeable meaning. These polymers consists of mobile (motile), preferentially highly mobile (motile) chains, so the matrix is characterized by the absence of a solid surface as the attachment point for the stimulating agents such as antibodies or fragments thereof, and which is in strong contrast to currently used beads or microspheres which regularly have an inflexible, immobile, stiff surface. As a result the nanomatrix comprising a matrix of mobile polymer chains is flexible and adjustable to the form of the surface of the cells. In addition as a result the nanomatrix is a nanomatrix wherein the majority (i.e. more than 50%), preferentially more than 80 %, and more preferentially more than 90%, and most preferentially more than 99% of the total volume of the nanomatrix in aqueous solution consists of mobile polymer chains.

The matrix consists of a polymeric, preferentially biodegradable or biocompatible inert material that is non-toxic to cells. Preferentially the matrix is composed of hydrophilic polymer chains, which obtain maximal mobility in aqueous solution due to hydration of the chains. The mobile matrix is the only or at least main component of the nanomatrix regardless the agents which are attached thereto.

The mobile matrix may be of collagen, purified proteins, purified peptides, polysaccharides, glycosaminoglycans, or extracellular matrix compositions. A polysaccharide may include for example, cellulose ethers, starch, gum arabic, agarose, sepharose, dextran, chitosan, hyaluronic acid, pectins, xanthan, guar gum, starch, or alginate. Other polymers may include polyesters, polyethers, polyacrylates, polyacrylamides, polyamines, polyethylene imines, polyquaternium polymers, polyphosphazenes, polyvinylalcohols, polyvinylacetates, polyvinylpyrrolidones, block copolymers, or polyurethanes. Preferentially the mobile matrix is a polymer of dextran.

An agent may be attached or coupled to the mobile matrix by a variety of methods known and available in the art. The attachment may be covalent or non-covalent, electrostatic, or hydrophobic and may be accomplished by a variety of attachment means, including for example, chemical, mechanical, enzymatic, or other means whereby an agent is capable of stimulating the cells. For example, the antibody to a cell surface structure first may be attached to the matrix, or avidin or streptavidin may be attached to the matrix for binding to a biotinylated agent. The antibody to the cell surface structure may be attached to the matrix directly or indirectly, e.g. via an anti-isotype antibody. Another example includes using protein A or protein G, or other non-specific antibody binding molecules, attached to matrices to bind an antibody. Another example for an indirect linkage comprises a fusion protein of an antibody or fragment comprising a poly-histidine tag in combination with a particle carrying either an anti-histidine antibody or fragment thereof or a metal chelate group capable of binding the poly-histidine tag. Alternatively, the agent may be attached to the matrix by chemical means, such as cross-linking to the matrix.

The term "magnetic" in "magnetic particle" as used herein refers to all subtypes of magnetic particles which could be prepared with methods well known to the skilled person in the art, especially ferromagnetic particles, superparamagnetic particles and paramagnetic particles. "Ferromagnetic" materials are strongly susceptible to magnetic fields and are capable of retaining magnetic properties when the field is removed. "Paramagnetic" materials have only a weak magnetic susceptibility and when the field is removed quickly lose their weak magnetism. "Superparamagnetic" materials are highly magnetically susceptible, i.e. they become strongly magnetic when placed in a magnetic field, but, like paramagnetic materials, rapidly lose their magnetism.

The term "label" as used herein refers to a detectable molecule attached directly or indirectly to a target, and which can be used to, for example detect, trace, separate, isolate, deplete, or quantify the target. Targets may be single cells or cell populations, cell surface structures, carbohydrates, proteins, and peptides. Examples for labels comprise fluorescent, magnetic, and isotopic labels. In some cases, the label can be a part of the target itself.

The term "reversible tagging of cells" as used herein refers to a method in which cells are firstly tagged using a tag such as a label or a particle. In some cases, a combination of tags may be used. The cells then may be processed, for example cultivated, activated, expanded, sorted, isolated, or analyzed. Afterwards, the tag is removed from the cells. In the case of antibodies and fragments thereof, the tag may be removed or dissolved by allowing a monovalent binding of said antibodies or fragments thereof to their antigen at that step, resulting in a loss of multivalent binding and a dissociation of antigen and tagged antibody due to the kinetic properties of the antibodies or fragments thereof.

The terms "closed cell sample processing system" and ""closed and sterile (cell culture) system" can be used interchangeably. The term "closed cell sample processing system" as used herein refers to any closed system which reduces the risk of cell culture contamination while performing culturing processes such as the introduction of new material, e.g. transformation and transduction, and performing cell culturing steps such as proliferation, differentiation, activation, and/or separation of cells. Such a system allows to operate under GMP or GMP-like conditions ("sterile") resulting in cell compositions which are clinically applicable. Herein exemplarily the CliniMACS Prodigy^{®} (Miltenyi Biotec GmbH, Germany) is used as a closed cell sample processing system. This system is disclosed in WO2009/072003. It is not intended to restrict the use of the method of the present invention to the CliniMACS^{®} Prodigy.

### Embodiments

The antibody fragment of the invention may by be presented in various embodiments as disclosed herein and described in detail in the preceding sections. Common to all these embodiments of the humanized antibody fragment specific for the antigen CD3 is the existence of the sequence of SEQ ID NO: 16 and SEQ ID NO:17, in the humanized heavy and light chain variable domains, respectively.

In one present disclosure, an antibody or fragment thereof has CDR sequences (e.g., an IMGT, Chothia, or Kabat CDR) that differ from those of the parental antibody clone OKT3. CDR sequences that differ from those of murine parental antibody clone include amino acid changes, such as substitutions of 1 or 2 amino acids if a CDR is 3-4 amino acids in length, or substitutions of 1, 2, 3, or 4 amino acids if a CDR is 5-7 amino acids in length, or substitutions of 1, 2, 3, 4, 5, 6, or 7 of amino acids in the sequence of a CDR if a CDR is 10 amino acids or greater in length. The amino acid that is substituted can have similar charge, hydrophobicity, or stereochemical characteristics. In some disclosures, the amino acid substitution(s) is a conservative substitution. In other disclosures, the amino acid substitution(s) is a non-conservative substitution. Such substitutions are within the ordinary skill of an artisan. The antibody or antibody fragments thereof that contain the substituted CDRs can be screened to identify antibodies binding to CD3 antigen.

In one disclosure a humanized antibody or fragment thereof as disclosed herein may be used for enrichment (positive selection) of CD3-expressing (CD3+) cells from a sample comprising CD3-expressing cells. In a preferred embodiment, the sample comprises CD3-expressing cells and CD3 non-expressing cells (other cells). Methods suited for enrichment are well known in the art and include but are not limited to flow cytometry such as FACS^{®} or magnetic cell separation such as MACS@. The enriched CD3-expressing cells may be used in further various analyses such as cytokine expression or receptor signaling or may be used in a pharmaceutical composition comprising these CD3-expressing cells. The enriched CD3-expressing cells may be expanded *in vitro* to a therapeutic effective amount prior to application to a patient in need thereof.

In one disclosure a humanized antibody or fragment thereof as disclosed herein may be used for depletion of CD3-expressing cells from a sample comprising CD3-expressing cells. In a preferred embodiment, the sample comprises CD3-expressing cells and CD3 non-expressing cells (other cells). Methods suited for depletion are well known in the art and include but are not limited to flow cytometry such as FACS^{®} or magnetic cell separation such as MACS^{®}.

Exemplarily the principle of MACS@ separation (Miltenyi Biotec GmbH, Germany) is described here: Antibodies or fragments thereof specific for CD3 antigen can be used for direct or indirect magnetic labeling of lymphocyte subsets. CD3 antigen is expressed on T cells and thymocytes. First the CD3-expressing cells are magnetically labeled with CD3 MicroBeads, i.e. CD3 antibodies or fragments thereof conjugated to magnetic particles. Then the cell suspension is loaded on a MACS@ Column which is placed in the magnetic field of a MACS@ Separator. The magnetically labeled CD3-expressing cells are retained on the column. The unlabeled cells run through, this cell fraction is depleted of CD3+ cells. After removal of the column from the magnetic field, the magnetically retained CD3+ cells can be eluted as the positively selected cell fraction.

Some examples of applications in the context of enrichment (positive selection) and depletion of cells using the antibodies or fragments thereof as disclosed herein might be: (a) Depletion of human CD3+ cells from peripheral blood or lymphoid tissue for allogeneic stem cell transplantation, or for preparation of T cells depleted grafts, or for subsequent enrichment of CD3 non-expressing cells (e.g. NK cells without NKT cells); (b) positive selection of human CD3+ cells from peripheral blood, lymphoid tissue, or tissue homogenate (such as tumor tissue) for analytical purposes, for subsequent enrichment, or for adoptive cell transfer therapy (e.g. donor leukocyte infusion or gene-modified T cells, respectively allogeneic or autologous transfers).

For positive selection of CD3+ T cell subsets the antibody or fragment thereof specific for CD3 can be combined for example with antibodies or fragments thereof specific for antigens such as CD4, CD8, CD25, CD27, CD28, CD45RA, CD45R0, CD56, CD62L, CD95, CCR7, CD127, CD137, CD279, Tim-3, and/or LAG-3. In one embodiment, the antibody or fragment thereof specific for CD3 can be combined for example with Annexin V protein and/or with antibodies or fragments thereof specific the surface antigen CD19, and/or CD14, and/or CD56, in order to ensure that only live CD3+ T cells have been isolated.

In one disclosure a humanized antibody or fragment thereof specific for CD3 as disclosed herein may be used for the positive selection of naive regulatory T cells for clinical applications. Using peripheral blood or lymphoid tissue, the humanized CD3 antibody or fragment thereof is combined with antibodies or fragments thereof specific for CD4, CD25, CD45RA, and CD127 in order to isolate naive regulatory T cells (characterized by CD3+, CD4+, CD25 hi, CD45RA+, CD127dim).

For a combined depletion of CD3+ T cell and other cells the antibody or fragment thereof specific for CD3 can be combined for example with antibodies or fragments thereof specific for antigens such as CD4, CD8, CD11b, CD11c, CD14, CD15, CD16, CD19, CD34, CD38, CD43, CD44, CD56, CD133, and/or CD271.

In one disclosure, also cells other than T cells, but also having a T cell receptor or an antigen receptor, may be stimulated by an antibody or fragment thereof specific for CD3 alone or in combination with an antibody or fragment thereof specific for CD28. In a preferred embodiment, these cells express chimeric antigen receptors (CARs) or exogenous T cell receptors (TCRs). Examples for cells expressing CARs and/or TCRs comprise T cells, dendritic cells, mesenchymal stem cells, NKT cells, and NK cells. Examples for target molecules of CARs and/or TCRs comprise the cell surface markers CD19, CD20, and CD22.

In one embodiment of the invention, T cells are stimulated by anti-CD3 antibody fragment as disclosed herein alone or in combination with anti-CD28 antibody or fragment thereof as disclosed herein, for example to increase the transduction efficiency (for example lentiviral transduction). Antibody fragments specific for CD3 and specific for CD28, respectively, may be both linked to particles.

In one disclosure, a closed cell sample processing system such as the CliniMACS Prodigy^{®} (Miltenyi Biotec) is used to deplete CD3+ T cells. For example a leukapharesis of 8E+09 total cells is connected by sterile welding to a tubing set fitted onto the ClinicMACS Prodigy^{®}. The cells are washed and labeled with antibodies or fragments thereof specific for CD3 coupled to magnetic particles. Labeled T cells are specifically and automatically isolated from the rest of the cells by magnetic enrichment using a column (that is part of the tubing set) fitted into a controllable magnetic field. The isolated T cells can either be enriched (positive fraction) or discarded (negative fraction) and further used for clinical applications.

In one disclosure a humanized antibody or fragment thereof specific for the CD3 antigen as disclosed herein may be coupled to a tag. Said tag may be a magnetic particle or a fluorophore. In one disclosure a humanized antibody or fragment thereof specific for CD3 as disclosed herein may be used for the fluorescent labeling of cells expressing CD3. This may be accomplished *in vitro* (e.g. in cell culture) or *in vivo* (e.g. in the body). The fluorescent label may be attached to the antibody or fragment thereof directly (e.g. by chemical conjugation) or indirectly (e.g. by biotinylation of the humanized antibody or fragment thereof and binding of the biotinylated antibody to a streptavidin fluorochrome conjugate). In some variants said humanized antibody or fragment thereof specific for CD3 may be used in combination with a humanized antibody or fragment thereof specific for CD28 as disclosed herein.

In one disclosure a humanized antibody or fragment thereof as disclosed herein may be used for the identification and monitoring of CD3+ T cells *in vitro* or *in vivo.* In some variants said humanized antibody or fragment thereof specific for CD3 may be used in combination with a humanized antibody or fragment thereof specific for CD28 as disclosed herein.

In one disclosure, a humanized antibody or fragment thereof as disclosed herein may be used for the reversible tagging of cells. Said antibody or fragments thereof may be conjugated to a label, for example a fluorescent dye or a particle. Cells are tagged by the multivalent binding of said antibody or fragments thereof which is coupled to the tag. The tag is removed from cells by switching from multivalent to monovalent binding of said antibody or fragments thereof. The dissociation of said antibody or fragments thereof bound to the antigen depends on suitable kinetic properties such as K_{D} (equilibrium dissociation constant) and/or K_{off} ("off rate constant") values, allowing monovalently bound antibodies or fragments to be released from the cells. Methods to switch from multivalent to monovalent binding of said antibody or fragments thereof comprise the removal of an antibody or fragment thereof from a tag such as a particle or a label for example by enzymatic cleavage using a specific cleavage site or structure, or by competitive displacement of the tag from the antibody or fragment thereof by addition of an excess of a substance able to displace either the tag or the antibody or fragment thereof (e.g. biotin which displaces an anti-biotin antibody fluorochrome conjugate bound to a biotinylated antibody or fragment thereof directed against a target antigen such as CD3, wherein the biotinylated antibody or fragment is cross-linked by the anti-biotin antibody fluorochrome conjugate).

The cell compositions enriched and/or separated by the use of antibodies or fragments thereof as disclosed herein may be administered either alone, or as a pharmaceutical composition in combination with diluents and/or with other components such cytokines. Briefly, pharmaceutical compositions of the present invention may comprise a cell composition enriched and/or separated by use of an antibody or fragment thereof as disclosed herein, in combination with one or more pharmaceutically or physiologically acceptable carriers, diluents or excipients. Such compositions may comprise buffers such as neutral buffered saline, phosphate buffered saline (PBS) and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

Preferentially, the compositions of the present disclosure are formulated for intravenous administration. The administration of cell compositions to the subject may be carried out in any convenient manner known in the art.

Pharmaceutical compositions of the present disclosure may be administered in a manner appropriate to the disease to be treated. Appropriate dosages may be determined by clinical trials. But the quantity and frequency of administration will also be determined and influenced by such factors as the condition of the patient, and the type and severity of the patient's disease.

### Examples

### Example 1: In silico aggregation analysis of parental murine antibodies OKT3 and 15E8.

The amino acid sequences of heavy and light chain variable domains of anti-CD3 antibody clone OKT3 and anti-CD28 antibody clone 15E8 were analyzed using algorithms known in the art to predict sequence regions which are prone to aggregation. It is known that such regions may interfere with the recombinant expression of a protein which results in low protein expression levels. Useful algorithms comprises for example PASTA (Walsh et al. (2014) Nucleic Acids Res. 42, W301-307), WALTZ (Maurer-Stroh et al. (2010) Nature Methods 7, 237-242), and AGGRESACN (Conchillo-Solé et al. (2007) BMC Bioinformatics 8, 65-81). The algorithms typically assign a numerical score to each amino acid, wherein the score is an indicator for the aggregation potential. FIG 1 shows the assignment of such a score to the heavy chain (of SEQ ID NO:13, FIG 1A) and light chain (of SEQ ID NO:14, FIG 1B) variable domains of murine parental CD3 antibody clone OKT3 and the heavy chain (of SEQ ID NO:18, FIG 1C) and light chain (of SEQ ID NO:19, FIG 1D) variable domains of murine parental anti-CD28 antibody clone 15E8. A score of 0 indicates no, a score of about > 20 indicates a moderate and a score of about > 50 indicates a high tendency to form aggregates. In the case of anti-CD3 OKT3, no aggregation-prone regions were detected. It was therefore expected that the recombinant expression of anti-CD3 antibody clone OKT3 would not be hampered by protein aggregation, and therefore normal expression levels and product titers were expected. In the case of anti-CD28 15E8, the light chain is predicted to have an increased risk of aggregation, which may result in lower expression levels and product titers. Humanized variants of anti-CD28, as presented in table 2, are also predicted to have an increased risk of aggregation, at least as strong as the parental anti-CD28 sequence.

### Example 2: Humanization of anti-CD28 antibody clone 15E8 by CDR grafting into a human germline framework sequence.

The CDRs and the numbering of the amino acid residues according to the IMGT nomenclature were determined by submitting the heavy and light chain sequences of the parental murine anti-CD28 antibody clone 15E8 to the IMGT "DomainGapAlign" tool. The resulting numbering schemes and CDR definitions are depicted in FIG 2A and FIG 2B, and the CDRs are also described in SEQ ID NO:7 to SEQ ID NO:12. In a next step, the amino acid sequences of the light and heavy chains of the parental murine anti-CD28 antibody clone 15E8 were compared against reference databases of human germline V regions (polypeptide sequences). The current versions (August 2014) of the IMGT human IG reference directory sets were used as reference databases. These sequences include FR1, CDR1, FR2, CDR2, and FR3, but not CDR3 and FR4. CDR3 of the heavy chain is primarily assembled from a small part of a V segment, the whole D segment and a small part of a J segment. CDR3 of the light chain is primarily assembled from a small part of a V segment and a small part of a J segment. FR4 is derived from the J segment. Prior to further processing, pseudogenes and duplicate sequences were removed from the IMGT reference sets. For the identification of human germline sequences with high identity to the framework regions of the original non-human heavy and light chain variable domains, the identity of framework regions FR1, FR2, and FR3, and of CDR1 and CDR2 were compared at the amino acid level against the processed IMGT reference sets by using the Smith-Waterman algorithm implemented in the ssearch36 program which is part of the FASTA36 package (http://faculty.virginia.edu/wrpearson/fasta/). For the heavy chain, the allele IGHV4-4*08 of the V segment (SEQ ID NO:27) was selected as suitable template for CDR grafting. For the light chain variable domain, the allele IGKV4-1*01 of the V segment (SEQ ID NO:28) was selected as suitable template for CDR grafting. For FR-4, the alleles IGHJ4*01 (SEQ ID NO:33) for the heavy chain and IGKJ4*01 (SEQ ID NO:34) for the light chain variable domain were used as templates. Additionally, the anti-CD28 antibody sequences were compared against a combined sequence database comprising the IMGT reference set, the NCBI germline sequences and V-BASE using the abYsis web server (http://www.bioinf.org.uk/abysis/index.html). The selection of the above-described template sequences was confirmed that way. Finally, the CDRs (according to the IMGT definition) of the selected human germline template sequences IGHV4-4*08 and IGKV4-1*01 were replaced by the CDRs of the parental murine anti-CD28 antibody clone 15E8 (SEQ ID NO:7-12), resulting in "anti-CD28graft VH_final_IMGT" (SEQ ID NO:29) and "anti-CD28graft VL_final_IMGT" (SEQ ID NO:30).

### Example 3: Structure modeling of humanized antibody variants specific for CD28 and identification of amino acid residues for backmutation.

In order to identify amino acid residues in the framework regions FR1, FR2, and FR3 of the CDR-grafted human germline sequences (SEQ ID NO:29 and SEQ ID NO:30) which differ from the parental murine anti-CD28 antibody clone 15E8 (SEQ ID NO:18 and SEQ ID NO:19), pairwise alignments between these sequences were generated using the LALIGN program. Amino acid residues within the framework regions which physically interact (by hydrogen bonds, electrostatic, hydrophobic, or other interactions) with amino acid residues within the CDR regions ("Vernier residues") and which are different between the parental murine anti-CD28 antibody clone 15E8 and the human template germline sequence may be critical for antigen binding and structural integrity of the humanized antibody. Those residues have to be backmutated, i.e. the respective amino acid residue within the CDR-grafted sequence has to be replaced by the corresponding amino acid at the same position within the parental murine anti-CD28 antibody clone 15E8. The identification of amino acid residues suitable for backmutation requires knowledge about the three-dimensional structure of the antibody to be modified. As no structural data for the parental murine anti-CD28 antibody clone 15E8 was available, an alternative antibody with a high sequence identity to the parental murine anti-CD28 antibody clone 15E8 was used. For that purpose, BLAST searches of light and heavy chain sequences of 15E8 against the PDB protein database (http://www.pdb.org) were conducted, and structures with corresponding peptide sequences that show the highest bit score and the lowest E-values were selected separately for light and heavy chain. For the heavy chain, the structure 1GIG (chain A) and for the light chain, the structure 4BKL (chain C) was selected, respectively. For the structure-based analysis the publicly available software "Discovery Studio 4.0" (Accelrys) was used. Firstly, the CDRs according to the IMGT nomenclature were defined within the structure and all framework atoms within a radius of 5Å of the CDRs were identified. Subsequently, each framework amino acid identified by this procedure was checked for its conservation between the human template and the parental murine anti-CD28 antibody clone 15E8. Using this procedure, amino acids specifically interacting with the CDRs and probably stabilizing their structure were identified and backmutated in the corresponding human CDR grafted germline sequence. Four different humanized variants were designed: "huCD28_v1" comprising SEQ ID NO:20 and 21, "huCD28_v2" comprising SEQ ID NO:22 and 23, "huCD28_v3" comprising SEQ ID NO:24 and 25, and "huCD28_v4" comprising SEQ ID NO:26 and 25. The main difference between huCD28_v3 and huCD28_v4 versus huCD28_v1 and huCD28_v2 are two backmutations in the light chain variable domains, namely the amino acid substitutions D74A and S93P (IMGT nomenclature).

### Example 4: Cloning of DNA vectors and transformation of E. coli.

For the expression of non-humanized and humanized Fab fragments specific for CD3 and CD28, respectively in *E.coli,* synthetic genes encoding for non-humanized and humanized heavy chain and light chain variable domains (designated as VH and VL) of either anti-CD3 clone OKT3 (SEQ ID NO:13 in combination with SEQ ID NO:14; SEQ ID NO:15 in combination with SEQ ID NO:14; SEQ ID NO:16 in combination with SEQ ID NO:17) or of anti-CD28 clone 15E8 (SEQ ID NO:18 in combination with SEQ ID NO:19; SEQ ID NO:20 in combination with SEQ ID NO:21; SEQ ID NO:22 in combination with SEQ ID NO:23; SEQ ID NO:24 in combination with SEQ ID NO:25; SEQ ID NO:26 in combination with SEQ ID NO:25), respectively, for human kappa light chain constant region (designated as CL, SEQ ID NO:32), and for human IgG1 heavy chain constant region CH1 (SEQ ID NO:31) were designed using a standard software tool. Compatible flanking restriction sites were added to the synthetic genes sequences to facilitate direct cloning into an *E.coli* expression vector. Gene synthesis was performed externally by a service provider. The gene sequence encoding for the light chain (LC) variable domain (VL and CL) of the Fab fragment comprises a phoA signal sequence for periplasmatic translocation of the light chain. The gene sequence encoding for the heavy chain (HC) variable domain (VH and CH1, optionally followed by a poly-histidine tag) of the Fab fragment comprises a pelB signal sequence for periplasmatic translocation of the heavy chain. Cloning was performed using standard techniques known in the art. The resulting *E.coli* expression vector is shown in FIG 3. All vector regions affected by the cloning procedure were validated by DNA sequencing.

### Example 5: Expression of Fab fragments in E.coli.

For production of Fabs specific for CD3 and specific for CD28, *E.coli* W3110 cells were transformed with an appropriate expression vector such as, for example, "pOPE313 huCD3_mut" which encodes for the humanized Fab variant "huCD3_mut" with heavy chain (VH) and light variable (VL) domains as described in SEQ ID NO:16 and SEQ ID NO:17. Pre-cultures of transformed *E.coli* were grown in complex media (containing soy-peptone, yeast extract and NaCl, supplemented with glucose) in shake flasks over night at 37°C. The next morning, a stirrable lab-scale bioreactor (3 L) containing complex media (containing soy-peptone, yeast extract and NaCl, supplemented with glucose as carbon source) was inoculated to a final optical density at 600 nm of 0.05 using the pre-culture. The cells were grown at 28°C and pH 7.0 with a pO₂ of at least 30%. Before induction the bioreactor was cooled to 25°C and target protein expression was subsequently induced by addition of 0.2 mM IPTG. The cultivation was continued for at least 20 h at 25°C. Glucose was constantly added as carbon source, and pH was kept at 7.0. Cell harvest was performed by centrifugation at 6000×g for 1 hour. Harvested cell pellets were stored at -20°C for further processing.

### Example 6: Purification of Fab fragments.

Recombinant Fabs specific for CD3 and for CD28 were purified from fermented *E.coli* cells of example 5 by a periplasmic extraction step followed by chromatographic purification steps. For cell extraction, frozen *E.coli* pellets were resuspended in 10 mL of periplasmic extraction buffer (100 mM Tris, 10 mM EDTA) per 1 g cell pellet (wet weight) and incubated at 37°C with constant agitation for at least 16 hours. Afterwards the suspension was centrifuged at 6000×g for 1 hour. The supernatant contained the periplasmic fraction of *E.coli,* harboring the Fabs. Cell pellets were discarded. The supernatant was filtered and applied to a suitable chromatography column packed with an IMAC resin (for example ProCatchHis Resin, Miltenyi Biotec) for metal chelate affinity chromatography on an FPLC system (ÄKTA) for a first affinity capture purification step. Impurities were washed away using imidazolecontaining buffer (5 mM). The target protein was eluted using a buffer containing an increased imidazole concentration of at least 50 mM. Eluted fractions were pooled and applied to a cation exchange matrix (for example SP sepharose). Elution was performed using a phosphate (25 mM) based buffer in the pH range of 6.0-7.4 containing NaCl in the range of 0.3-0.5 M. Eluates containing Fabs were pooled and the protein content was quantified by BCA assay and UV absorption (A₂₈₀ nm). Aliquots of purified Fabs specific for CD3 and of purified Fabs specific for CD28 were stored at -70°C towards use. In some cases, Fabs were rebuffered into, for example, PBS/EDTA. FIG 4 shows a Coomassie-stained SDS gel with samples of different steps of the purification process of Fab variant "huCD3_mut" as an example. Tables 1 and 2 list the amount of purified Fabs from 100 g *E.coli* cell pellet. Because the expression und purification process was nearly identical for all variants, the amount of purified Fabs is a good indicator for the individual expression level of a Fab variant. For CD3, the highest amount of protein after purification was found for the humanized Fab variant "huCD3_mut", which shows that the expression level of variant "huCD3_mut" was also the highest of all tested variants. For CD28, the highest amount of protein after purification was found for the humanized Fab variant "huCD28_v3", which shows that the expression level of variant "huCD28_v3" was also the highest of all tested variants.

**Table 1**

| **Variant** | **SEQ ID NO** | **yield (mg)** | **yield (%)** |
|---|---|---|---|
| chCD3 Fab (chimeric) | 13 + 14 | 0.5 | 100 |
| chCD3_mut Fab (chimeric) | 15 + 14 | 20 | 4000 |
| huCD3_mut Fab (humanized) | 16 + 17 | 36 | 7200 |

Table 1 shows the yield of purified Fab variants specific for CD3 antigen (from 100 g *E.coli* cell pellet). The yield of Fab variant "chCD3" was set to 100%. The SEQ ID NO refer to the sequences of the heavy and the light chain variable domains, respectively.

**Table 2**

| **Variant** | **SEQ ID NO** | **yield (mg)** | **yield (%)** |
|---|---|---|---|
| chCD28 Fab (chimeric) | 18 + 19 | 1 | 100 |
| huCD28_v1 Fab (humanized) | 20 + 21 | 0.05 | 5 |
| huCD28_v2 Fab (humanized) | 22 + 23 | 0.02 | 2 |
| huCD28_v3 Fab (humanized) | 24 + 25 | 11 | 1100 |
| huCD28_v4 Fab (humanized) | 26 + 25 | 9 | 900 |

Table 2 shows the yield of purified Fab variants specific for CD28 antigen (from 100 g *E.coli* cell pellet). The yield of Fab variant "chCD28" was set to 100%. The SEQ ID NO refer to the sequences of the heavy and the light chain variable domains, respectively.

### Example 7: Determination of kinetic constants of Fab variants using recombinant CD28 antigen.

The Fab variants of example 6 specific for CD28 antigen were analyzed by surface plasmon resonance spectroscopy (Biacore). Therefore, recombinant CD28 antigen (extracellular domain, expressed in mammalian cells) was immobilized on a CM5 chip by standard amine coupling. The Fab variants were used as analytes in HBS-EP buffer (0.01 M HEPES pH 7.4, 0.15 M NaCl, 3 mM EDTA, 0.005% v/v Surfactant P20) in a concentration of 50 µg/mL. Evaluation was conducted with the BIAeval Software using an 1:1 binding mass transfer model. Table 3 shows the determined kinetic constants: the rate constants "k₍ₒₙ₎" and "k_{(off)}" and the equilibrium dissociation constant "K_{D}" for the five Fab variants.

**Table 3**

| **Variant** | **k₍ₒₙ₎ [1/Ms]** | **k_{(off)} [1/s]** | **K_{D} [M]** | **affinity [%]** |
|---|---|---|---|---|
| chCD28 Fab | 1.0E+06 | 3.2E-02 | 3.2E-08 | 100 |
| huCD28_v1 Fab | 1.9E+06 | 8.0E-02 | 4.2E-08 | 76 |
| huCD28_v2 Fab | 0.6E+06 | 5.5E-02 | 9.2E-08 | 35 |
| huCD28_v3 Fab | 0.5E+06 | 2.6E-02 | 5.2E-08 | 62 |
| huCD28_v4 Fab | 0.4E+06 | 2.1E-02 | 5.3E-08 | 60 |

Table 3 shows the kinetic constants of purified Fab variants. The affinity, determined by the K_{D} value in [M], of Fab variant "chCD28" towards CD28 antigen was set to 100%. M, molar; s, second.

### Example 8: Staining of cells expressing CD3 or CD28 antigen.

The anti-CD3 Fab variants of example 6 were analyzed for binding to and staining of CD3+ cells. PBMC (peripheral blood monocular cells) were incubated with 2.5 µg/mL of either Fab variant chCD3_mut or humCD3_mut. An OKT3-PE conjugate (1.5 µg/mL) served as positive control. Fab bound to cells was detected after incubation with an anti-polyhistidine-PE conjugate. Cells stained only by an anti-polyhistidine-PE conjugate without further incubation by a Fab served as negative control. FIG 5A-D show the dot plots of the flow cytometry analysis for CD3 variants. A similar analysis was conducted for anti-CD28 Fab variants of example 6 for binding/staining CD28+ cells. PBMC (peripheral blood monocular cells) were incubated with 5 µg/mL of either Fab variant huCD28_v1, huCD28_v2, huCD28_v3, or huCD28_v4. A 15E8-PE conjugate (3.0 µg/mL) served as positive control. In addition, cells were incubated with an anti-CD3-FITC conjugate. Fab bound to cells was detected after incubation with an anti-polyhistidine-PE conjugate. Cells stained only by an anti-polyhistidine-PE conjugate without further incubation by a Fab served as negative control. FIG 5E-J show the dot plots of the flow cytometry analysis for CD28 variants. All Fab variants are functional, showing a similar staining pattern as their murine parental antibodies.

### Example 9: Generation of nanomatrices comprising anti-CD3 Fabs, parental OKT3 antibody, anti-CD28 Fabs, and parental 15E8 antibody.

Fab variants of example 6 and purified parental murine antibodies specific for CD3 and specific for CD28, respectively, were used for the generation of nanomatrices. Dextran was used as a mobile polymer chain. In this example, magnetic nanomatrices were produced, and therefore a modified procedure of Molday and MacKenzie was used. Ten grams of Dextran T40 (GE Healthcare), 1.5 g FeCl₃ 6 H₂O and 0.64 g FeCl₂ 4 H₂O are dissolved in 20 ml H₂O, and heated to 40° C. While stirring, 10 ml 4 N NaOH are added slowly and the solution is heated to 70° C for 5 min. The particle suspension is neutralized with acetic acid. To remove aggregates the suspension is centrifuged for 10 min at 2,000×g and filtrated through a 0.22 µm pore-size filter (Millex G V, Millipore). Unbound Dextran is removed by washing in a high-gradient magnetic field (HGMF). HGMF washing of magnetic nanomatrices is performed in steelwool columns made as described below and placed in a magnetic field of approx. 0.6 Tesla (MACS permanent magnet, Miltenyi Biotec). Ten milliliters of nanomatrix suspension are applied to a 15×40 mm column of 2 g steelwool. The loaded column is washed with 30 ml 0.05 M sodium acetate. After removing the column from the external magnetic field, the magnetic nanomatrices are eluted with 0.05 M sodium acetate. The nanomatrices form a brown suspension. The relative particle concentration is given as optical density at 450 nm. The size of the nanomatrices was determined by electron microscopy and dynamic light scattering to be 30±20 nm (e.m.) and 65±20 nm (DLS). The nanomatrices show superparamagnetic behavior, as determined by susceptibility measurements. The size of the trapped ferrit microcrystals was determined from magnetic measurements to be approximately 10 nm. Antibodies and Fabs were conjugated to separate nanomatrices by standard bioconjugation chemistry (Bioconjugate Techniques, 2nd Edition, By Greg T. Hermanson, Published by Academic Press, Inc., 2008). After sterile filtration, the resulting nanomatrices were stored in PBS buffer/0.3% Poloxamer 188 at -70°C. As a result the following nanomatrices were generated: A nanomatrix comprising parental anti-CD3 antibody OKT8, a nanomatrix comprising parental anti-CD28 antibody 15E8, a nanomatrix comprising anti-CD3 Fab variant chCD3_mut, a nanomatrix comprising anti-CD3 Fab variant huCD3_mut, a nanomatrix comprising anti-CD28 Fab variant huCD_v3, and a nanomatrix comprising anti-CD28 Fab variant huCD3_v4.

### Example 10: Stimulation of T cells by nanomatrices comprising an anti-CD3 protein alone or in combination with nanomatrices comprising an anti-CD28 protein.

Nanomatrices comprising antibody and Fab variants of example 9 were used for the stimulation of T cells. Therefore, Pan T cells were isolated from PBMC using the Pan T Cell Isolation Kit, human (Miltenyi Biotec). Afterwards, the cells were labeled with carboxyfluorescein succinimidyl ester (CFSE) and cultured at a density of 1E+06 cells per cm² in TexMACS GMP Medium (Miltenyi Biotec) supplemented with 200 IU/mL MACS GMP IL-2 (Miltenyi Biotec). A nanomatrix comprising either an anti-CD3 Fab variant or the full-length antibody (OKT3) was used alone or in different combinations with a nanomatrix comprising an anti-CD28 Fab variant or the full-length antibody (15E8), respectively. The final concentration during T cell stimulation was 500 ng protein/mL for each anti-CD3 and anti-CD28 full length antibodies and 333 ng protein/mL for each anti-CD3 and anti-CD28 Fab variants, respectively. The stimulation capacity of the nanomatrices was determined after 48 hours of cell culture using flow-cytometry for the detection of the early activation markers CD25 and CD69 (among viable T cells). Cells were afterwards cultured for further 5 days, and the proliferation rate (among viable cells) was determined via flow-cytometric acquisition. FIG 6A shows the frequency of T cells expressing both CD25 and CD69 after stimulation, and FIG 6B shows the proliferation rate of stimulated T cells. Each dot represents the sample of one donor, wherein the horizontal line represents the median values. For anti-CD3 alone, a nanomatrix comprising humanized Fab huCD3_mut led to a higher number of cells expressing early activation markers and also a higher proliferation rate than a nanomatrix comprising parental OKT3 antibody. As expected, nanomatrices comprising an anti-CD28 protein used without a nanomatrix comprising an anti-CD3 protein did not result in a detectable expression of CD25 and CD69 or increased cell proliferation. In combination with a nanomatrix comprising anti-CD28 antibody 15E8, a nanomatrix comprising huCD3_mut Fab resulted in a higher activation and a similar proliferation rate compared to a nanomatrix comprising OKT3 antibody. In combination with a nanomatrix comprising anti-CD3 antibody OKT3, both nanomatrices comprising either huCD28_v3 or huCD28_v4 Fab showed a higher activation and a higher proliferation rate than a nanomatrix comprising 15E8 antibody, and these results were very robust and reproducible. The combination of a nanomatrix comprising Fab variant huCD3_mut with a nanomatrix comprising either Fab variant huCD28_v3 or huCD28_v4 gave the best result in terms of robust induction of high frequency of activated cells plus high proliferation rate.

### Example 11: Aggregation and dimer formation of murine parental OKT3 and 15E8 antibodies.

Purified OKT3 (anti-CD3) and 15E8 (anti-CD28) antibodies at concentrations of 1.0 mg/mL and 0.2 mg/mL, respectively, were stored in closed glass vials over a period of 26 weeks at 25 °C and 40% relative humidity in a climatic chamber. At several time points, samples of the stored antibodies were analyzed by analytical SEC-HPLC to differentiate between monomeric, dimeric and aggregated antibodies. As depicted in FIG 7A, there is only minor aggregate formation at the beginning of storage in the case of 15E8. For OKT3, a linear to exponential aggregate formation was detected, reaching 1% aggregate content after 26 weeks. A similar trend can be seen in the formation of antibody dimers (i.e. a dimer of two antibodies): while for 15E8 (FIG 7B) the dimer content remains stable in the range of 3-5% during storage, the dimer content OKT3 successively increased up to 9% after 26 weeks. At that time point, the content of OKT3 monomer decreased to less than 90%.

### Example 12: Stability of purified anti-CD3 Fabs.

Fab variants of example 6 at a concentration of 0.2 mg/mL in PBS/ETDA buffer were incubated at 37 °C for one week. PBMC was stained with these Fabs as well as with non-incubated Fabs (reference) using a titer of 1 µg/mL. Fab bound to cells was detected after incubation with an anti-polyhistidine-PE conjugate. Exemplary dot plots are shown for chCD3 Fab (FIG 8B+C) and huCD3_mut Fab (FIG 8D+E). FIG 8B+D represent non-incubated (unstressed) Fabs, and FIG 8C+E represent incubated (stressed) Fabs. The median fluorescence intensity (MFI, in %) of stained cells is illustrated in FIG8A. For non-incubated Fabs, each MFI was set to 100%. It can be seen that the MFI of the non-CDR-mutated variant is reduced by 35% after thermal stressing of the Fab, while the decrease of the MFI values of both CDR-mutated variants (humanized as well as not-humanized) is much less (11-12%). The Fab molecules, incubated and non-incubated, were also analyzed by non-reducing SDS-PAGE. No differences could be detected, all samples showed the same protein band without any visible change after thermal stressing. There were no visible protein aggregates or degradation products.

### Example 13: Stability of a nanomatrices comprising anti-CD3 proteins.

To test the stability of nanomatrices comprising anti-CD3 proteins, nanomatrices of example 9 were incubated at 37 °C for one week. Afterwards, these incubated samples as well as non-incubated reference samples were used for the stimulation of T cells as described in example 10. Again, the expression of both early activation markers CD25 and CD69 after 48 hours of stimulation and the proliferation rate of T cells after one week of stimulation were analyzed. FIG 9 illustrates the increased stability of nanomatrices comprising either Fab variant chCD3_mut or huCD3_mut compared to a nanomatrix comprising murine parental OKT3 antibody: both the expression of early activation markers and the proliferation rate of T cells are decreased in the case of a nanomatrix comprising OKT3 after thermal stressing, while both nanomatrices comprising one of the Fab variants are much less affected after thermal stressing. The nanomatrix comprising Fab variant huCD3_mut shows the best results, with no or only minimal loss in the capability to efficiently stimulate T cells.

### Example 14: Reversible tagging of cells with anti-CD3 and anti-CD28 Fabs.

Fab variants of example 6 were used for the reversible tagging of cells. Therefore, 1 µg/ml of Fab variant huCD3_mut, huCD28_v3, or huCD28_v4, respectively, was incubated with PBMC in phosphate/EDTA buffer for 10 min at 4 °C. Afterwards, the sample was split. One part was incubated for 10 min at 4 °C with an anti-polyhistidine-APC conjugate (Miltenyi Biotec, in a titer according to the manufacturer's instructions) which is able to bind to the Fab variants. Phosphate/EDTA buffer was added, and the tagged cells were centrifuged at 300×g for 5 min and then analyzed by flow cytometry. The other part was firstly washed with phosphate/EDTA buffer, and then incubated with an anti-polyhistidine-APC conjugate and analyzed by flow cytometry as described above. FIG 10A and 10B show the dot plots of the flow cytometry analysis for anti-CD3 variants, and FIG 10C-10F the dot plots for anti-CD28 variants. All Fab variants are able to tag cells (FIG 10A, 10C, and 10E), wherein the tag comprises the anti-polyhistidine fluorochrome conjugate. The tagging is reversible and can be removed by washing the cells (FIG 10B, 10D, and 10F). The results also shows that the kinetic properties of the Fabs are in a useful range for specific and reversible tagging of cells.

## Claims

1. An *in-vitro* method for polyclonal stimulation of T cells comprising contacting a population of T cells with a humanized anti-CD3 antibody fragment, wherein said antibody fragment comprises a heavy chain variable domain comprising the sequence of SEQ ID NO: 16 , and a light chain variable domain comprising the sequence of SEQ ID NO: 17, and wherein said anti-CD3 antibody fragment is linked to particles.

2. The method according to claim 1, wherein said population of T cells is additionally contacted with a humanized anti-CD28 antibody fragment, wherein said anti-CD28 antibody fragment comprises a heavy chain variable domain comprising the sequence of SEQ ID NO:26 or SEQ ID NO:24, and a light chain variable domain comprising the sequence of SEQ ID NO: 25, and wherein said anti-CD28 antibody fragment is linked to the same or to separate particles regarding the particles to which the anti-CD3 antibody fragment is linked.

3. The method according to claim 1 or 2 wherein said particles are beads with a solid phase surface ranging in size between 500 nm to 10 µm.

4. The method according to any one of claims 1 to 3, wherein said method is performed in a closed and sterile cell culture system.

## Patentansprüche

1. *In-vitro*-Verfahren zur polyklonalen Stimulation von T-Zellen, umfassend das In-Kontakt-Bringen einer Population von T-Zellen mit einem humanisierten Anti-CD3-Antikörperfragment, wobei das Antikörperfragment eine variable Domäne der schweren Kette, die die Sequenz von SEQ ID NO:16 umfasst, und eine variable Domäne der leichten Kette, die die Sequenz von SEQ ID NO:17 umfasst, umfasst, und wobei das Anti-CD3-Antikörperfragment an Partikel gebunden ist.

2. Verfahren nach Anspruch 1, wobei die Population von T-Zellen zusätzlich mit einem humanisierten Anti-CD28-Antikörperfragment in Kontakt gebracht wird, wobei das Anti-CD28-Antikörperfragment eine variable Domäne der schweren Kette, die die Sequenz von SEQ ID NO:26 oder SEQ ID NO:24 umfasst, und eine variable Domäne der leichten Kette, die die Sequenz von SEQ ID NO:25 umfasst, umfasst, und wobei das Anti-CD28-Antikörperfragment an dieselben Partikel gebunden ist, an die das Anti-CD3-Antikörperfragment gebunden ist, oder an andere.

3. Verfahren nach Anspruch 1 oder 2, wobei es sich bei den Partikeln um Kügelchen mit einer Festphasenoberfläche in einer Größe im Bereich zwischen 500 nm und 10 µm handelt.

4. Verfahren nach einem der Ansprüche 1 bis 3, wobei das Verfahren in einem geschlossenen und sterilen Zellkultursystem durchgeführt wird.

## Revendications

1. Procédé *in vitro* de stimulation polyclonale de lymphocytes T comprenant la mise en contact d'une population de lymphocytes T avec un fragment d'anticorps anti-CD3 humanisé, dans lequel ledit fragment d'anticorps comprend un domaine variable de chaîne lourde comprenant la séquence de SEQ ID n° 16 et un domaine variable de chaîne légère comprenant la séquence de SEQ ID n° 17 et ledit fragment d'anticorps anti-CD3 étant lié à des particules.

2. Procédé selon la revendication 1, dans lequel ladite population de lymphocytes T est de plus mise en contact avec un fragment d'anticorps anti-CD28 humanisé, ledit fragment d'anticorps anti-CD28 comprenant un domaine variable de chaîne lourde comprenant la séquence de SEQ ID n° 26 ou de SEQ ID n° 24 et un domaine variable de chaîne légère comprenant la séquence de SEQ ID n° 25 et ledit fragment d'anticorps anti-CD28 étant lié aux mêmes particules ou à des particules distinctes concernant les particules auxquelles le fragment d'anticorps anti-CD3 est lié.

3. Procédé selon la revendication 1 ou 2 dans lequel lesdites particules sont des billes avec une surface de phase solide se situant dans la plage en taille comprise entre 500 nm et 10 □m.

4. Procédé selon l'une quelconque des revendications 1 à 3, dans lequel ledit procédé est effectué dans un système de culture cellulaire fermé et stérile.
